# EUROPEAN PATENT APPLICATION

(11) **EP 4 477 650 A1**
(43) Date of publication of application: **18.12.2024**
(21) Application number: 23179741.6
(22) Date of filing: 16.06.2023
(51) Int. Cl.: C07D 279/00, C07D 293/00, C07D 279/08, C07D 293/10

(54) **METHOD FOR PRODUCING AROMATIC THIAZINES OR SELENAZINES**

(71) Applicant: Universität Heidelberg, 69117 Heidelberg (DE)
(72) Inventor: MASTALERZ, Michael, 68526 Ladenburg (DE); HOLSTEN, Mattes, 69115 Heidelberg (DE); NAUMANN, Laura, 69117 Heidelberg (DE)
(74) Representative: Müller-Boré & Partner Patentanwälte PartG mbB

(57) **Abstract**

The present invention relates to production methods as well as the use of hexamethylenetetramine and an acid for converting a thiol or selenol group and an adjacent CH group in an aromatic group into a 2-H-[1,3]-thiazine or 2-H-[1,3]-selenazine.

## Description

The present invention relates to a method for producing aromatic thiazines and selenazines as well as to a method for producing pharmaceutical products based on said aromatic thiazines and selenazines. In addition, the present invention relates to the use of hexamethylenetetramine and an acid for converting a thiol or selenol group and an adjacent CH group in an aromatic group into a 2-H-[1 ,3]-thiazine or 2-H-[1 ,3]-selenazine.

Since the beginning of the 21st century, the benzothiazine scaffold became more and more interesting for agricultural use as bactericides or medical applications like cell death inhibitors, anti-tuberculosis agents and arthritis as well as HIV medications. One of these medically active compounds is PD 404182 having the following formula (4) which was first developed by Birck et al., J. Am. Chem. Soc. 2000, 122, 9334-9335, as 3-deoxy-d-manno-octulosonic acid 8-phosphate (KDO 8-P) synthase inhibitor.

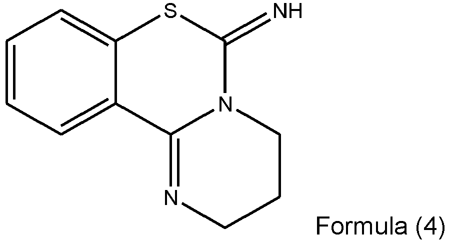

Later, Mizuhara et al., Org. Biomol. Chem. 2012, 10, 6792-6802, found that it also showed potent anti-HIV activities. They synthesized it using copper-mediated carbon disulphide addition followed by hydrolysis and nitrogen introduction using cyanogen bromide, which are highly toxic compounds.

2H-Benzo[e][1,3]thiazine derivatives were already synthesized in the 1980s, predominantly by using strong Lewis acids like antimony(V) chloride or phosphorous(V) oxychloride. However, the respective reactions are very water sensitive and have limited substrate scope. Additionally, the corresponding reactions involve several steps, since thiophenol compounds first have to be converted into chloromethyl thioether or amidomethyl ether derivatives before cyclisation. Furthermore, benzoselenazines are also of interest.

Thus, the technical problem underlying the present invention is to provide novel means for obtaining aromatic thiazine and selenazine derivatives and corresponding pharmaceutical products based on said aromatic thiazines and selenazines, which do not involve several reaction steps and avoid toxic and/or sensitive compounds.

The solution to the above technical problem is achieved by the embodiments characterized in the claims.

In particular, the present invention relates to a method for producing a compound represented by the following general formula (1), 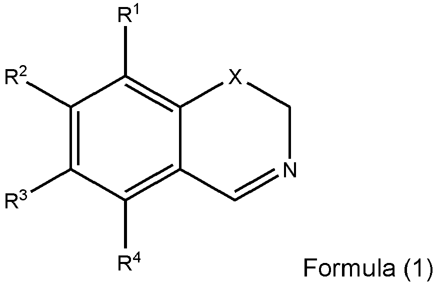 wherein the method comprises reacting a compound represented by the following general formula (2) 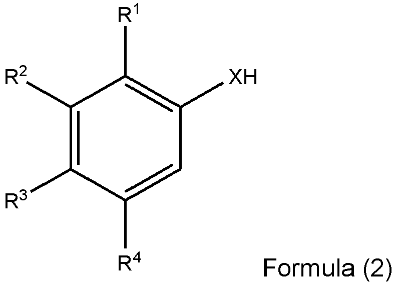
with hexamethylenetetramine (HMTA) in the presence of an acid,
wherein X is S or Se, and
R¹ to R⁴ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, a halogen atom, -XH, -S-alkyl, -NE¹E², -NO₂, -CN, -OE³, -C(O)E⁴, -C(O)NE⁵E⁶, -COOE⁷, and -SO₃E⁸, wherein E¹ to E⁸ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, wherein two or more of R¹ to R⁴ may bind to each other to form one or more rings. That is, the method for producing the compound of formula (1) according to the present invention relates to a one step reaction in which the compound of formula (2) is reacted with hexamethylenetetramine (HMTA) in the presence of an acid.

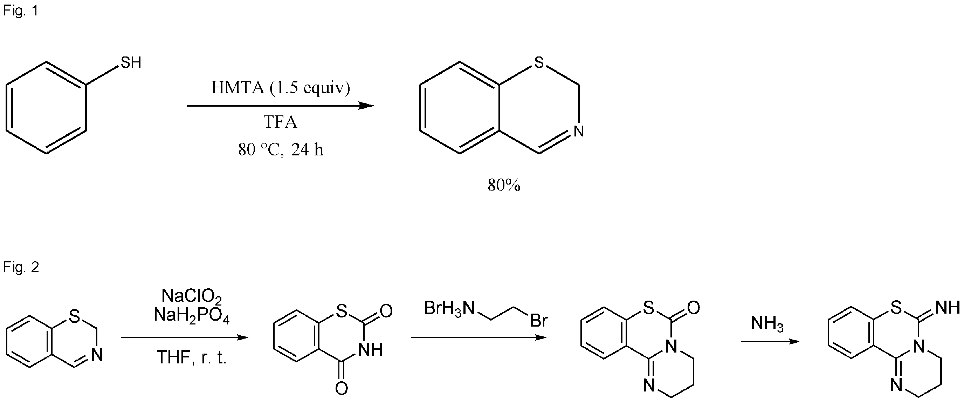

When applying the method of the present invention, it is advantageously possible to obtain aromatic thiazine and selenazine derivatives, in particular 2H-benzo[e][1 ,3]thiazine derivatives, in a one-step transformation using cheap and benign compounds. Furthermore, an extensive substrate scope and increased yields can preferably be realized. An exemplary reaction is depicted in Fig. 1.

If not stated otherwise, the following definitions apply to the terms "halogen", "alkyl group", "cycloalkyl group", "alkenyl group", "cycloalkenyl group", "alkynyl group", "aryl group", and "heteroaryl group". Herein the term "halogen" refers particularly to fluorine atoms, chlorine atoms, bromine atoms, and iodine atoms, preferably fluorine atoms and bromine atoms. The term "alkyl group" refers particularly to a branched or linear alkyl group having 1 to 20, preferably 1 to 12, more preferably 1 to 6, and most preferably 1 to 4 carbon atoms, which can be substituted or unsubstituted. Examples of alkyl groups represent methyl groups, ethyl groups, propyl groups, isopropyl groups, butyl groups, isobutyl groups, tert-butyl groups, pentyl groups, hexyl groups, and heptyl groups. The term "cycloalkyl group" refers particularly to a cycloalkyl group having 3 to 10, preferably 4 to 8, more preferably 5 or 6, and most preferably 6 carbon atoms, which can be substituted or unsubstituted.

Examples of cycloalkyl groups represent cyclobutyl groups, cyclopentyl groups, and cyclohexyl groups. The term "alkenyl group" refers particularly to a branched or linear alkenyl group having 2 to 20, preferably 2 to 12, more preferably 2 to 6, and most preferably 2 to 4 carbon atoms, which can be substituted or unsubstituted. Examples of alkenyl groups represent vinyl groups and allyl groups. The term "cycloalkenyl group" refers particularly to a cycloalkenyl group having 4 to 10, preferably 5 to 8, more preferably 5 or 6, and most preferably 6 carbon atoms, which can be substituted or unsubstituted. Examples of cycloalkenyl groups represent cyclopentenyl groups, cyclopentadienyl groups, cyclohexyl groups, and cyclohexadienyl groups. The term "alkynyl group" refers particularly to a branched or linear alkynyl group having 2 to 20, preferably 2 to 12, more preferably 2 to 6, and most preferably 2 to 4 carbon atoms, which can be substituted or unsubstituted. Examples of alkynyl groups represent ethynyl groups, 1-propynyl groups, and propargyl groups. The term "aryl group" refers particularly to an aryl group consisting of 1 to 6, preferably 1 to 4, more preferably 1 to 3 aromatic rings, and most preferably 1 ring, which can be substituted or unsubstituted. Examples of aryl groups represent phenyl groups, anthracenyl or naphthyl groups. The term "heteroaryl group" refers particularly to a heteroaryl group consisting of 1 to 6, preferably 1 to 4, more preferably 1 to 3 aromatic rings including heteroatoms, which can be substituted or unsubstituted. Heteroatoms, which are present in heteroaryl groups are for example N, O and S. Examples of heteroaryl groups represent pyridyl groups, pyrimidinyl groups, thienyl groups, furyl groups or pyrrolyl groups.

According to the present invention, the alkyl groups, the cycloalkyl groups, the alkenyl groups, the cycloalkenyl groups, the alkynyl groups, the aryl groups and the heteroaryl groups may be substituted or unsubstituted. The potential substituents are not specifically limited. Accordingly, instead of hydrogen atoms any substituent known in the prior art can be bonded to the further positions of the corresponding groups. For example, the potential substituents may be selected from the group consisting of a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms, a halogen atom, -NL¹L², -NO₂, -CN, -OL³, -C(O)L⁴, -C(O)NL⁵L⁶, -COOL⁷, and -SO₃L⁸, wherein L¹ to L⁸ are each independently selected from a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms. Accordingly, examples of substituted alkyl groups are aralkyl groups or alkyl groups substituted with e.g. halogen atoms, such as e.g. a trifluoromethyl group, or any other of the above-mentioned substituents. The term "aralkyl group" refers particularly to an alkyl group wherein one or more hydrogen atoms, preferably terminal hydrogen atoms of the alkyl chain, are replaced by aryl or heteroaryl groups. Examples of aralkyl groups represent benzyl groups or 1- or 2-phenylethyl groups. Preferably, the potential substituents are selected from the group consisting of a branched or linear alkyl group having 1 to 6 carbon atoms, a branched or linear alkenyl group having 2 to 6 carbon atoms, a branched or linear alkynyl group having 2 to 6 carbon atoms, a halogen atom, -NH₂, -NHCH₃, -N(CH₃)₂, -NO₂, -OH, -OCH₃, -OEt, -C(O)H, -C(O)CH₃, -C(O)Et, and -COOH. Moreover, one or more tetravalent carbon atoms (together with the hydrogen atoms bonded thereto), when present, in each of the alkyl groups, the cycloalkyl groups, the alkenyl groups, the cycloalkenyl groups, and the alkynyl groups may each independently be substituted by a member selected from the group consisting of O, (OCH₂CH₂)ₙO, S, (SCH₂CH₂)ₘS, C(O), C(O)O, NL⁹, and C(O)NL¹⁰, preferably O, (OCH₂CH₂)ₙO, C(O)O, and C(O)NL¹⁰, wherein n and m are each independently an integer from 1 to 6. Accordingly, for example an alkyl group may be interrupted by e.g. one or more PEG linkers and/or amide bonds, and an alkenyl group may contain a C(O) group, such as in an acryloyl group. The way the groups are introduced instead of a carbon atom is not specifically limited. For example, a carbon atom may be substituted by C(O)O in the sense of -C(O)O- or -OC(O)- and by C(O)NL¹⁰ in the sense of -C(O)NL¹⁰- or -NL¹⁰C(O)-. According to the present invention, L⁹ and L¹⁰ are each independently selected from the group consisting of a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms, -OG¹, -C(O)G², -C(O)NG³G⁴, -COOG⁵, and -SO₂G⁶. In a preferred embodiment, L⁹ and L¹⁰ are each independently selected from the group consisting of a hydrogen atom, a branched or linear alkyl group having 1 to 6 carbon atoms, an aryl group having 1 to 3 aromatic rings, -C(O)G², and -SO₂G⁶. Most preferably, L⁹ and L¹⁰ are each independently selected from the group consisting of a hydrogen atom and a branched or linear alkyl group having 1 to 6 carbon atoms. According to the present invention, G¹ to G⁶ are each independently selected from the group consisting of a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms. In a preferred embodiment, G¹ to G⁶ are each independently selected from the group consisting of a hydrogen atom, a branched or linear alkyl group having 1 to 6 carbon atoms, an aryl group having 1 to 3 aromatic rings.

If not stated otherwise, the alkyl groups, the cycloalkyl groups, the alkenyl groups, the cycloalkenyl groups, the alkynyl groups, the aryl groups, and the heteroaryl groups are preferably unsubstituted. Moreover, if not stated otherwise, the alkyl groups, the alkenyl groups, and the alkynyl groups are preferably linear.

According to the present invention, the compound represented by the general formula (2) has at least one -XH group. Accordingly, the compound represented by the general formula (2) may have more than one -XH group, e.g. one or more of R¹ to R⁴ can be a -XH group, which can all be converted by the reaction as shown in Fig. 1. In case one or more of R¹ to R⁴ are a -XH group, R² and/or R⁴ are preferably a -XH group, and more preferably R² and/or R⁴ are a -XH group and R¹ and/or R³ are a hydrogen atom. Preferably, the compound represented by the general formula (2) has (in total) one -XH group.

According to the present invention, X is S or Se. Preferably, X is S.

According to the present invention, R¹ to R⁴ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, a halogen atom, -XH, -S-alkyl, -NE¹E², -NO₂, -CN, -OE³, -C(O)E⁴, -C(O)NE⁵E⁶, -COOE⁷, and -SO₃E⁸. E¹ to E⁸ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, preferably E¹ to E⁸ are each independently selected from the group consisting of a hydrogen atom, a substituted or unsubstituted alkyl group, and a substituted or unsubstituted aryl group, most preferably E¹ to E⁸ are each independently selected from the group consisting of a hydrogen atom and a substituted or unsubstituted alkyl group. Preferably, R¹ to R⁴ are each independently selected from the group consisting of a hydrogen atom, a substituted or unsubstituted alkyl group, a substituted or unsubstituted alkenyl group, a halogen atom, -XH, -S-alkyl, -NO₂, -OE³, and -COOE⁷. More preferably, R¹ to R⁴ are each independently selected from the group consisting of a hydrogen atom, a substituted or unsubstituted alkyl group, -SMe, -OMe, -COOMe, and a halogen atom. Preferably, at least two, more preferably at least three of R¹ to R⁴ are a hydrogen atom.

In one preferred embodiment of the present invention each of R¹ to R⁴ is a hydrogen atom. In this embodiment, the compound represented by formula (1) is selected from the compound represented by the following formula (1a), preferably (1a) with X=S (i.e. 1a-S), and the compound represented by formula (2) is selected from the compound represented by the following formula (2a), preferably (2a) with X=S (i.e. 2a-S).

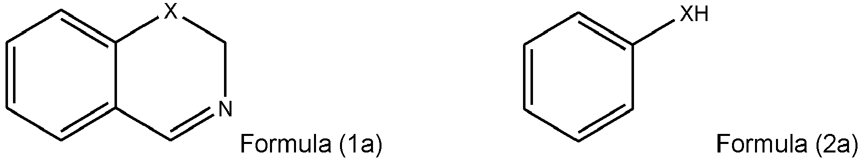

In another preferred embodiment of the present invention, each of R² to R⁴ is a hydrogen atom. In this embodiment, it is particularly preferred that R¹ is a substituted or unsubstituted, branched or linear alkyl group having from 1 to 6 carbon atoms, more preferably 1 to 4 carbon atoms, a halogen atom, -OE³, or -COOE⁷. More preferably, R¹ is selected from a methyl group, a bromine atom, -OMe, and -COOMe. Preferably, X is S. In this embodiment, the compound represented by formula (1) is preferably selected from the compounds represented by the following formulas (1b) to (1e), more preferably (1b) to (1e) with X=S (i.e. 1b-S to 1e-S), and the compound represented by formula (2) is preferably selected from the compounds represented by the following formulas (2b-S) to (2e-S), more preferably with X=S (i.e. 2b-S to 2e-S).

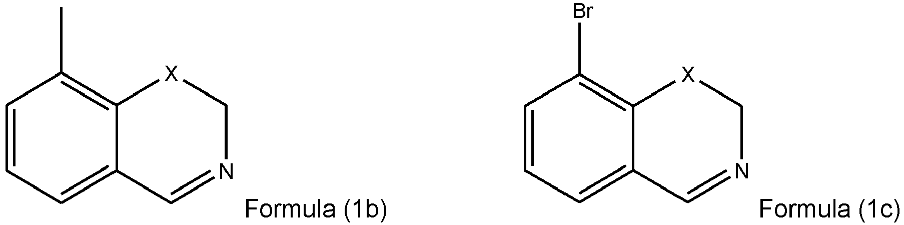

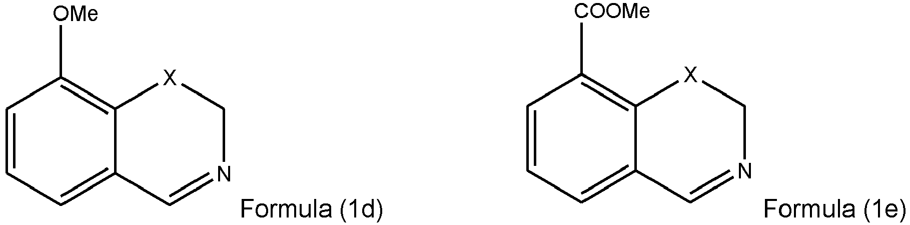

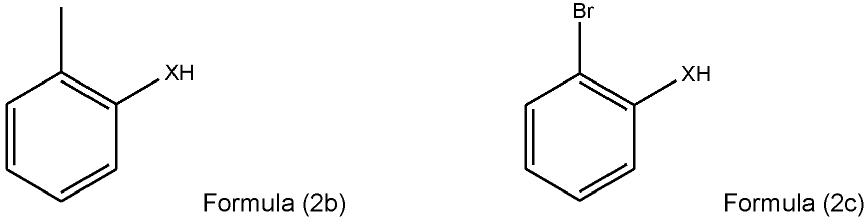

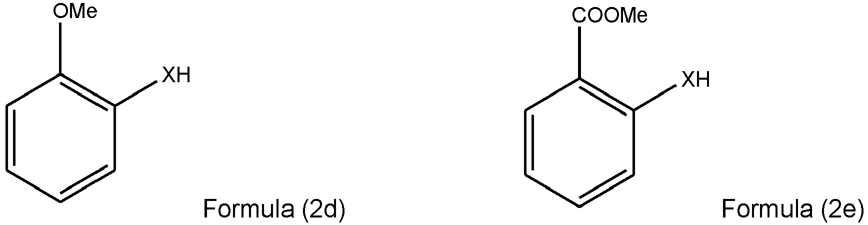

In another preferred embodiment of the present invention, each of R¹ and R³ is a hydrogen atom. In this embodiment, it is particularly preferred that one of R² and R⁴ is a hydrogen atom and the other one of R² and R⁴ is a substituted or unsubstituted, branched or linear alkyl group having from 1 to 6 carbon atoms, more preferably 1 to 4 carbon atoms, a halogen atom, -OE³, or -COOE⁷ More preferably, the other one of R² and R⁴ is selected from a methyl group, a bromine atom, a fluorine atom, and -OMe. In this context, it should be noted that irrespective of whether R² is a hydrogen atom or R⁴ is a hydrogen atom, the respective compound represented by formula (2) is identical (e.g. Formulas (2f) and (2g) below) and its respective reaction yields one or both of the corresponding compounds (1), wherein R² is a hydrogen atom (e.g. Formula (1g) below) or R⁴ is a hydrogen atom (e.g. Formula (1f) below). Preferably, X is S. In this embodiment, the compound represented by formula (1) is preferably selected from the compounds represented by the following formulas (1f) to (1m), more preferably (1f) to (1l) with X=S (i.e. 1f-S to 1l-S), and the compound represented by formula (2) is preferably selected from the compounds represented by the following formulas (2f) to (2m), more preferably (2f) to (2m) with X=S (i.e. 2f-S to 2m-S).

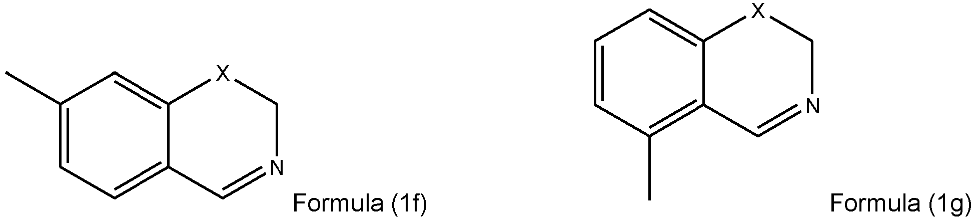

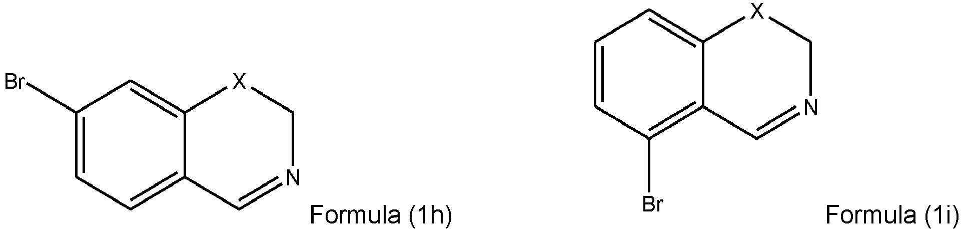

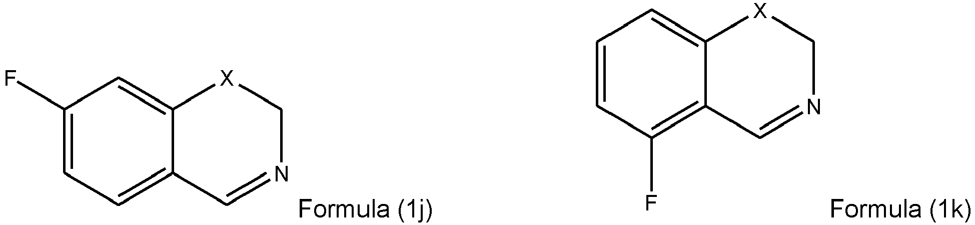

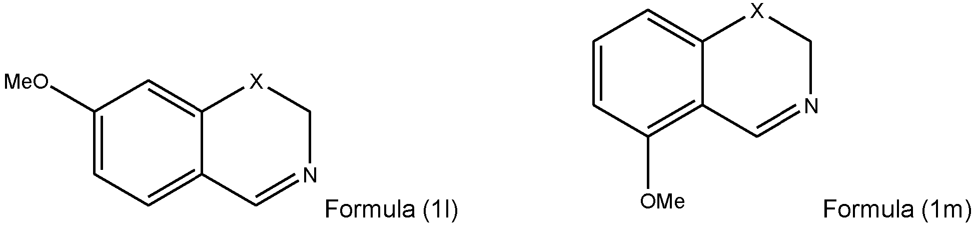

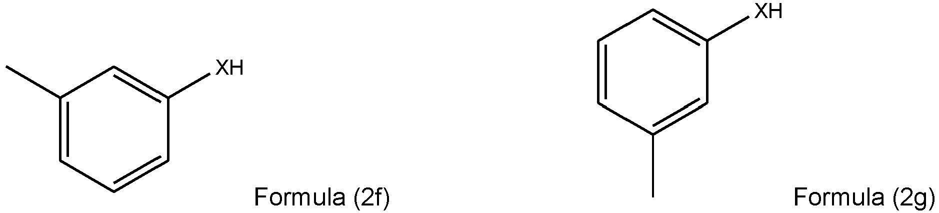

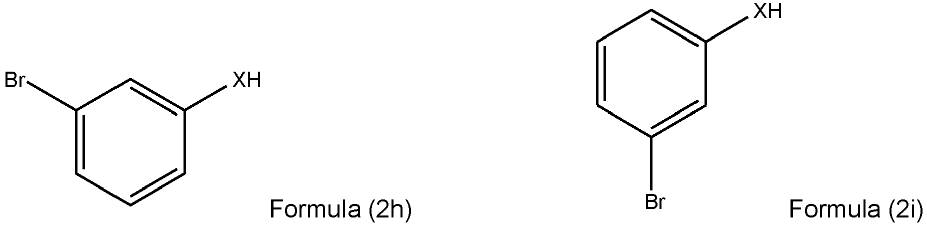

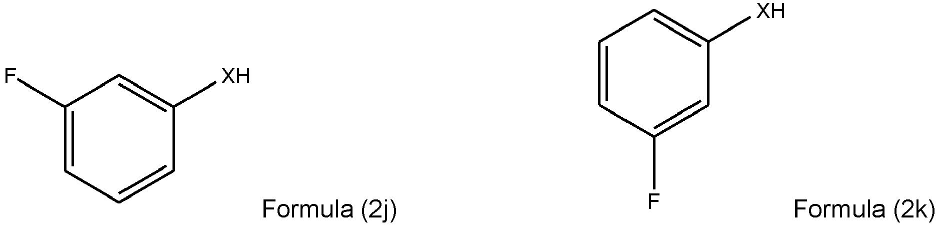

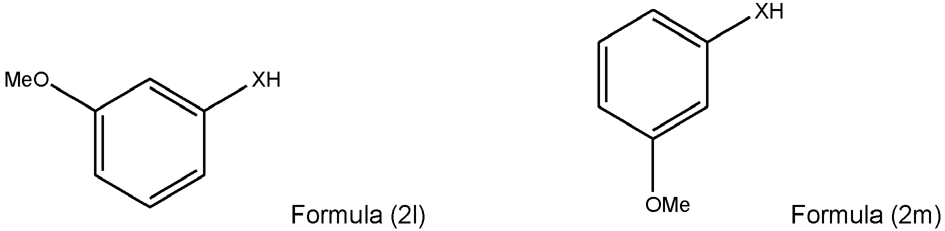

In another preferred embodiment of the present invention, each of R¹, R², and R⁴ is a hydrogen atom. In this embodiment, it is particularly preferred that R³ is a substituted or unsubstituted, branched or linear alkyl group having from 1 to 6 carbon atoms, more preferably 1 to 4 carbon atoms, a halogen atom, -S-alkyl, -OE³, -NO₂, or -COOE⁷. More preferably, R¹ selected from a methyl group, a tert-butyl group, a trifluoromethyl group, a bromine atom, a fluorine atom, -SMe, and -OMe. Preferably, X is S. In this embodiment, the compound represented by formula (1) is preferably selected from the compounds represented by the following formulas (1n) to (1t), more preferably (1n) to (1t) with X=S (i.e. 1n-S to 1t-S), and the compound represented by formula (2) is preferably selected from the compounds represented by the following formulas (2n) to (2t), more preferably (2n) to (2t) with X=S (i.e. 2n-S to 2t-S).

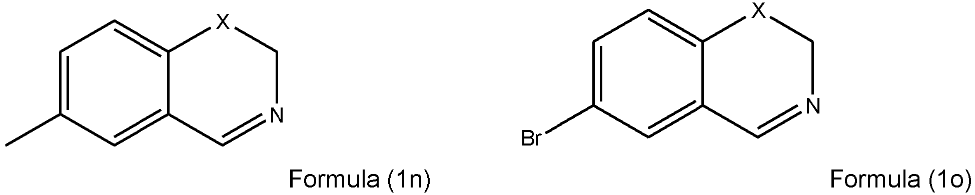

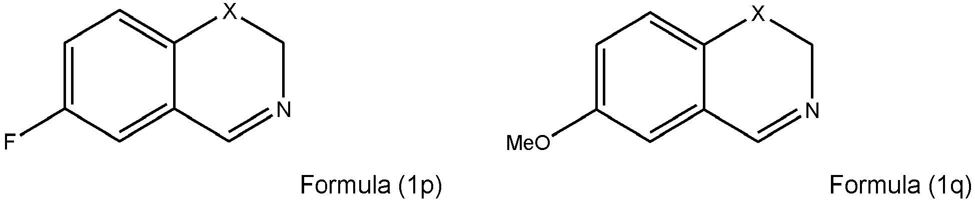

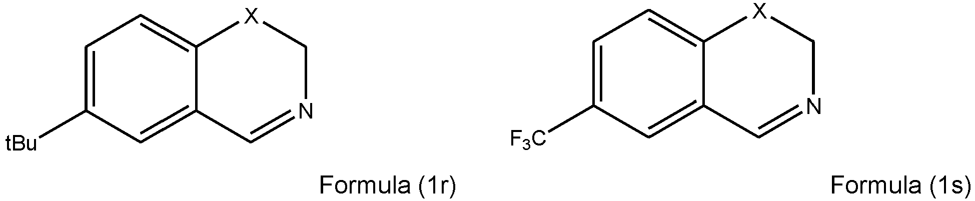

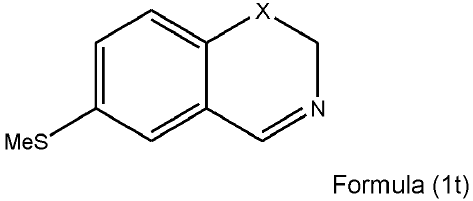

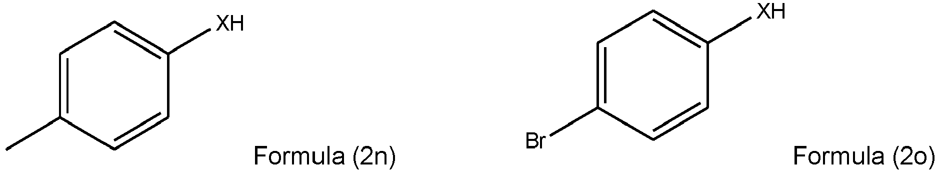

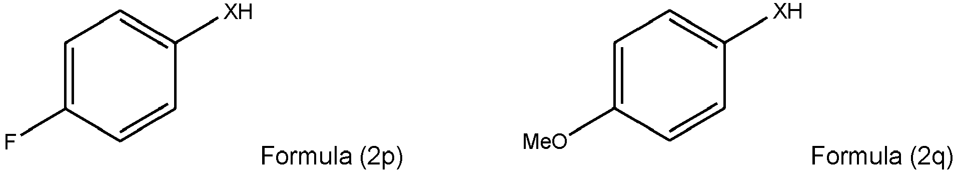

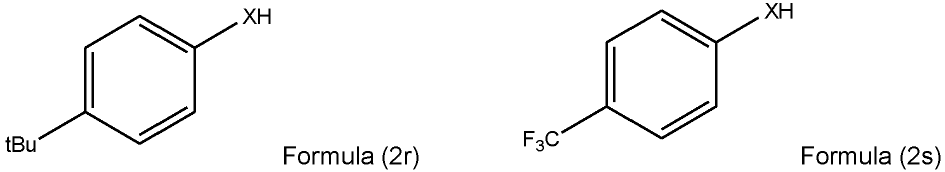

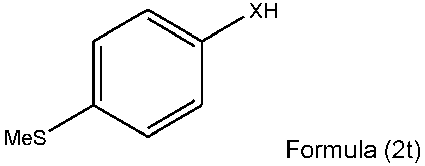

In another preferred embodiment of the present invention, each of R¹ and R⁴ is a hydrogen atom. In this embodiment, it is particularly preferred that R² and R³ are independently selected from the group consisting of a substituted or unsubstituted, branched or linear alkyl group having from 1 to 6 carbon atoms, more preferably 1 to 4 carbon atoms, a halogen atom, -OE³, and -COOE⁷. More preferably, R² and R³ are independently selected from a methyl group, a bromine atom, -OMe, and -COOMe. Most preferably, each of R² and R³ are -OMe. Preferably, X is S. In this embodiment, the compound represented by formula (1) is preferably the compound represented by the following formula (1u), more preferably (1u) with X=S (i.e. 1u-S), and the compound represented by formula (2) is preferably the compound represented by the following formula (2u), more preferably (2u) with X=S (i.e. 2u-S).

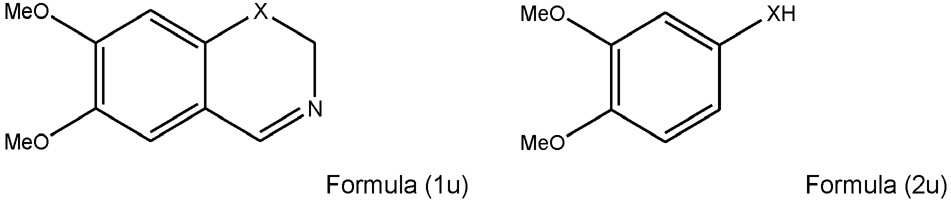

In another preferred embodiment of the present invention, two of R¹ to R⁴ bind to each other to form a ring. In this embodiment, it is particularly preferred that R¹ and R² or R³ and R⁴ bind to each other to form a ring, more preferably an aromatic ring, most preferably a six-membered aromatic ring. Moreover, the remaining of R¹ to R⁴ are each preferably a hydrogen atom. Preferably, X is S. In this embodiment, the compound represented by formula (1) is preferably selected from the compounds represented by the following formulas (1v) and (1w), more preferably (1v) and (1w) with X=S (i.e. 1v-S and 1w-S), and the compound represented by formula (2) is preferably selected from the compounds represented by the following formulas (2v) and (2w), more preferably (2v) and (2w) with X=S (i.e. 2v-S and 2w-S).

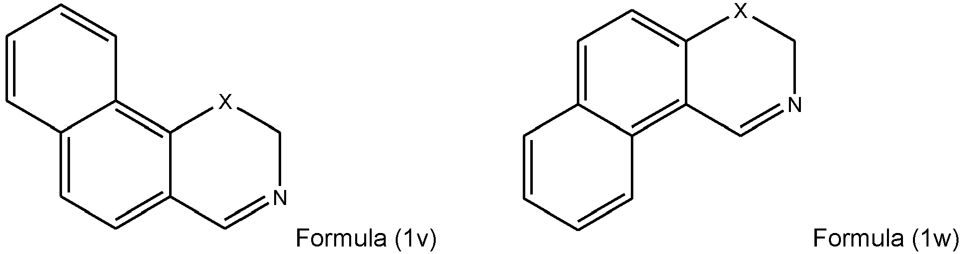

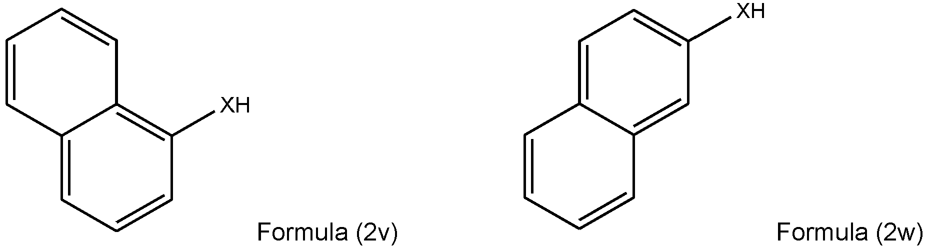

In another preferred embodiment of the present invention, one or more of R¹ to R⁴ are a -XH group. In this embodiment, it is particularly preferred that R² and/or R⁴ are a -XH group. Moreover, R¹ and/or R³ are preferably a hydrogen atom. Preferably, X is S. In this embodiment, the compound represented by formula (1) is preferably selected from the compounds represented by the following formulas (1x) and (1y), more preferably (1x) and (1y) with X=S (i.e. 1x-S and 1y-S), and the compound represented by formula (2) is preferably selected from the compounds represented by the following formulas (2x) and (2y), more preferably (2x) and (2y) with X=S (i.e. 2x-S and 2y-S).

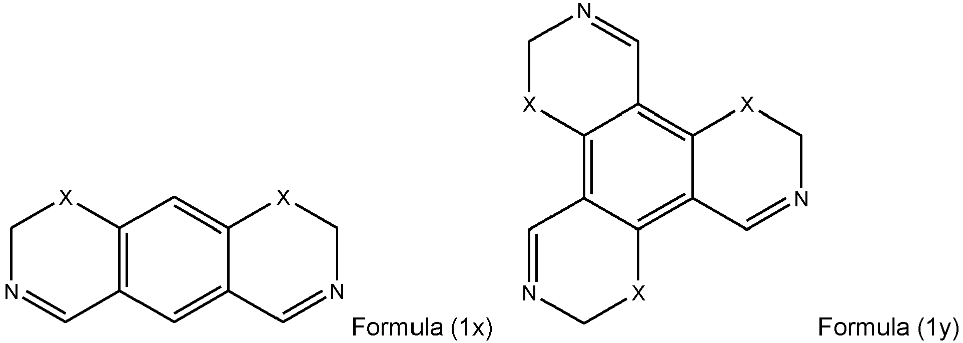

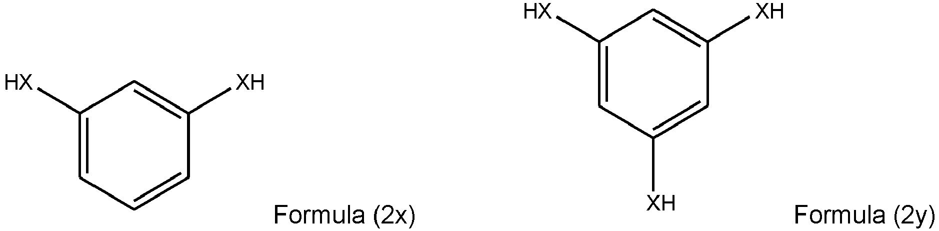

The above embodiments can be combined with each other without any particular limitation. The above statements and definitions given with respect to the specific embodiments analogously apply to each respective embodiment when combined with the other embodiments. In this embodiment, the compound represented by formula (1) is preferably selected from the compounds represented by the formulas (1a) to (1y), more preferably (1a) to (1y) with X=S (i.e. 1a-S to 1l-S and 1n-S to 1y-S), and the compound represented by formula (2) is preferably selected from the compounds represented by the following formulas (2a) to (2y), more preferably (2a) to (2y) with X=S (i.e. 2a-S to 2y-S).

According to the present invention, the method comprises reacting the compound represented by the general formula (2) with hexamethylenetetramine (HMTA) in the presence of an acid. Examples of acids, which can be used are trifluoroacetic acid, p-toluenesulfonic acid, trifluoromethanesulfonic acid, methanesulfonic acid, (non-fluorinated) carboxylic acids, such as acetic acid, and mineral acids. Said acids can be used alone or as a mixture of two or more thereof. Preferably, the acid is selected from trifluoroacetic acid, p-toluenesulfonic acid, trifluoromethanesulfonic acid, methanesulfonic acid, and acetic acid, more preferably from trifluoroacetic acid, p-toluenesulfonic acid, trifluoromethanesulfonic acid, and methanesulfonic acid. Most preferably, the acid is trifluoroacetic acid.

The acid can be present in an amount of at least 10 equivalents in relation to the amount of groups -XH present in the compound represented by the general formula (2). The acid is preferably present in an amount of at least 15 equivalents, more preferably at least 18 equivalents in relation to the amount of groups -XH present in the compound represented by the general formula (2). The upper limit of the amount of acid is not particularly limited and may for example be 100 equivalents in relation to the amount of groups -XH present in the compound represented by the general formula (2). The above amounts thereby indicate the added amounts of the component.

Hexamethylenetetramine can be present in an amount of at least 1.0 equivalents in relation to the amount of groups -XH present in the compound represented by the general formula (2). Hexamethylenetetramine is preferably present in an amount of at least 1.2 equivalents, more preferably at least 1.4 equivalents, most preferably at least 1.5 equivalents in relation to the amount of groups -XH present in the compound represented by the general formula (2). The upper limit of the amount of hexamethylenetetramine is not particularly limited and may for example be 5.0 equivalents in relation to the amount of groups -XH present in the compound represented by the general formula (2). The above amounts thereby indicate the added amounts of the component.

Moreover, the temperature at which the step of reacting the compound represented by the general formula (2) is carried out is not particularly limited. For example, the temperature may be at least 0°C, preferably at least 30°C, more preferably at least 50°C, and most preferably at least 70°C. The upper limit of the temperature is not particularly limited but may depend on the reactants used. For example, the upper limit of the temperature may be 140°C, preferably 110°C, and more preferably 90°C. Most preferably, the temperature at which the step of reacting the compound represented by the general formula (2) is carried out is 80°C.

Furthermore, the duration for which the step of reacting the compound represented by the general formula (2) is carried out is not particularly limited. For example, the duration may be from 30 s to 10 d, preferably from 5 min to 6 d, more preferably from 2 h to 4 d, and more preferably from 8 h to 2 d.

The step of reacting the compound represented by the general formula (2) may be carried out in the presence of an inert and/or dry atmosphere. For example, the step of reacting the compound represented by the general formula (2) can be carried out under nitrogen atmosphere or argon atmosphere, preferably under argon atmosphere.

In potential subsequent steps, the compound represented by the general formula (1) may be purified and isolated by various methods known in the art. For example, unreacted starting material and/or potential side-products may be removed by filtration, distillation, extraction, or column chromatography from the reaction mixture.

The isolated yield of the compound represented by the general formula (1) is not particularly limited. For example, the isolated yield of the compound represented by the general formula (1) in relation to the amount of the compound represented by the general formula (2) may be at least 10%. Preferably, the isolated yield of the compound represented by the general formula (1) in relation to the amount of the compound represented by the general formula (2) is at least 20%, more preferably at least 30%, more preferably at least 40%, more preferably at least 50%, more preferably at least 60%, more preferably at least 70%, and most preferably at least 80%.

In a further aspect, the present invention relates to a method for producing pharmaceutical products., i.e., a compound represented by the following general formula (3), 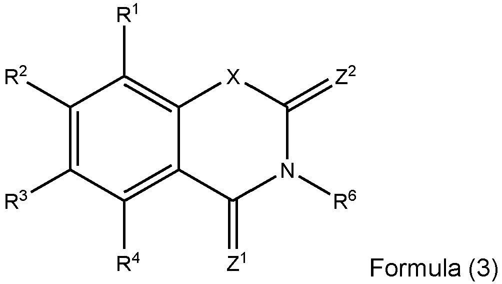 wherein the method comprises carrying out the production method according to the present invention to produce the compound of the general formula (1), and converting the compound of the general formula (1) into the compound of the general formula (3). In the general Formula (3), X and R¹ to R⁴ are defined as above and Z¹ and Z² are each independently selected from O or NR⁵, wherein R⁵ is selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group. In the above general Formula (3), R⁶ is selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, and a substituted or unsubstituted amino group, wherein R⁵ and R⁶ may bind to each other to form one or more rings.

According to a preferred embodiment, the compound of the general formula (3) is a compound of the formula (4)

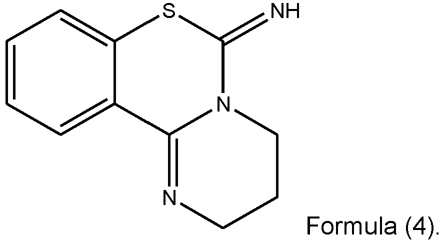

The compound of formular (4) may be obtained by the reaction scheme shown in Fig. 2. As can be taken from said reaction scheme, in contrast to the synthesis described by Mizuhara et al., using copper-mediated carbon disulphide addition followed by hydrolysis and nitrogen introduction using cyanogen bromide, according to the present invention, it is possible to refrain from using transition metals as well as highly toxic compounds like CS₂ as well as BrCN.

Thus, in a further aspect, the present invention relates to the use of hexamethylenetetramine and an acid for converting a thiol or selenol group and an adjacent CH group in an aromatic group into a 2-H-[1,3]-thiazine or 2-H-[1,3]-selenazine. The above statements and definitions analogously apply to this aspect of the present invention. In this context, "adjacent CH group" means a CH group directly bonded to the carbon atom to which the thiol or selenol group is bonded.
Fig. 1: Exemplary reaction scheme for producing a compound of general formula (1)
Fig. 2: Exemplary reaction scheme for producing a compound of general formula (3)

The present invention will be further illustrated in the following examples without being limited thereto.

### Experimental procedures:

### General

### Elemental analysis

The elemental analyses were performed by the microanalytical laboratory of University of Heidelberg using a *vario MICRO cube* device purchased from *Elementar.*

### IR Spectroscopy

Infrared spectroscopical measurements were conducted using a *Bruker Tensor 27 spectrometer* with ZnSe ATR crystal. The used abbreviations for describing the relative intensity of the obtained signals are vw (very weak, 0 - 10%), w (weak, 10 - 30%), m (medium, 30 - 60%), s (strong, 60 - 90%) and vs (very strong, 90 -100%).

### Mass Spectrometry

Mass spectrometric analyses were performed by the mass spectrometry department of University of Heidelberg under the supervision of Dr Jürgen H. Gross. The utilised devices were *Bruker AutoFlex Speed time-of-flight* spectrometer (MALDI-TOF), *Bruker ApexQe hybrid 9.4T FT-ICR* spectrometer (ESI), *JEOL AccuTOF GCx* spectrometer with direct insertion probe (EI) and *Bruker timsTOFfIeX* ion mobility-quadrupole-time-of-flight spectrometer with a trapped ion mobility spectrometry stage (MALDI-TOF, APCI). For the MALDI analyses DCTB (*trans*-2-[3-(4-tert-Butylphenyl)-2-methylpropenylidene)malononitrile) was used as matrix. High resolution MALDI-TOF spectra were obtained by utilizing an internal polyethylene glycole (PEG) standard.

### Melting Point Measurement

All measured melting points were obtained using a *Büchi Melting Point B-545* device.

### NMR Spectroscopy

Nuclear magnetic resonance spectra were obtained using a *Bruker Avance DRX 300* (300 MHz) or a *Bruker Avance III 400* (400 MHz) spectrometer. The chemical shifts *δ* are shown in parts per million (ppm) and the coupling constants *J* in Hertz (Hz). The spectra were recorded at 298 K, if not mentioned otherwise. The obtained signals of ¹H and ¹³C{¹H} NMR spectra were referred to known solvent signals (CDCl₃: ¹H NMR 7.26 ppm, ¹³C{¹H} NMR 77.2 ppm; DMSO-d₆: ¹H NMR 2.50 ppm, ¹³C{¹H} NMR 39.5 ppm; DCM-d₂: ¹H NMR 5.32 ppm, ¹³C{¹H} NMR 53.84 ppm; cf. G. R. Fulmer, A. J. M. Miller, N. H. Sherden, H. E. Gottlieb, A. Nudelman, B. M. Stoltz, J. E. Bercaw, K. I. Goldberg, Organometallics 2010, 29, 2176-2179.). Multiplicities were labelled with *s* (singlet), *d* (doublet), *dd* (doublet of doublet), *t* (triplet), *td* (triplet of doublet), *q* (quartet) and *m* (multiplet).

### Thin Layer and Flash Chromatography

Analytical thin layer chromatography was conducted using *TLC Silica gel 60 F₂₅₄* plates purchased from *Merck.* The resulted spots were analysed using ultraviolet radiation (254 nm and 366 nm). Flash chromatography was performed using *Silica gel 60 (40-63 µm*/ *230-400 mesh ASTM)* from *Machery-Nagel.*

### General procedure for synthesis of compounds represented by formula (1)

An 8 mL screw capped vial was filled with a thiophenol derivative (3 mmol if not mentioned otherwise) and hexamethylenetetramine (1.5 equiv. per thio group). Trifluoroacetic acid (5 mL per thio group) was added and the obtained solution was stirred at 80 °C for 24 h under argon atmosphere as shown in Fig. 1. After cooling down to room temperature the solution was neutralized with saturated aqueous sodium hydrogencarbonate solution. The mixture was extracted with dichloromethane (3 x 90 mL). The combined organic phases were washed with water (2 x 30 mL) and brine (30 mL) and dried using sodium sulphate. After filtration the solvent was evaporated *in vacuo* and the resulted residue was purified by flash chromatography.

### Example 1: 2H-Benzo[e][1,3]thiazine (1a-S)

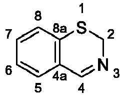

Reaction conditions based on 2.94 mmol benzenethiol (**2a-S**) (324 mg) were used. Compound **1a-S** was purified by flash chromatography using *n*-pentane/EtOAc (4:1 v/v) as eluent and obtained in 80% yield (350 mg, 2.34 mmol) as yellow oil.

**Retention factor:** 0.24 (*n*-pentane/EtOAc 4:1 v/v). **¹H NMR:** (300 MHz, DMSO-d₆) *δ* (ppm) 8.25 (t, *J* = 2.0 Hz, 1H, *H*4), 7.47 (dd, *J* = 7.4, 1.6 Hz, 1H, *H*5), 7.38 (td, *J* = 7.5, 1.6 Hz, 1H, *H*7), 7.33 - 7.20 (m, 2H, *H*6, *H*8), 4.78 (d, *J* = 1.9 Hz, 2H, *H*2). **¹³C{¹H} NMR:** (75 MHz, DMSO-d₆) *δ* (ppm) 160.0 (*C*4), 134.0 (*C*8a), 131.9 (*C*7), 130.1 (*C*5), 127.2 (*C*6/8), 126.7 (*C*4a), 126.1 (*C*6/8), 47.0 (*C*2). **HRMS (+EI):** [M]⁺: *m*/*z* calcd. for C₈H₇NS⁺: 149.02937, found: 149.02814. **IR:** (ATR, FT) *ṽ* (cm⁻¹) 3059 (vw), 3011 (vw), 2943 (vw), 2887 (vw), 2826 (vw), 1630 (s), 1585 (m), 1562 (w), 1466 (w), 1437 (w), 1425 (w), 1369 (w), 1294 (w), 1259 (w), 1211 (m), 1196 (m), 1159 (w), 1130 (vw), 1076 (m), 1032 (w), 945 (m), 920 (m), 854 (m), 754 (s), 737 (vs), 721 (s), 692 (m), 669 (m). **Elemental Anal.** Calcd. for C₈H₇NS: C, 64.40; H, 4.73; N, 9.39. Found: C, 64.30; H, 5.16; N, 9.79.

### Example 2: 2H-Benzo[e][1,3]selenazine (1a-Se)

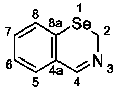

Reaction conditions based on 2.93 mmol selenophenol (**2a-Se**) (461 mg) were used. Compound **1a-Se** was purified by flash chromatography using DCM/MeOH (50:1 v/v) as eluent and obtained in 55% yield (319 mg, 1.63 mmol) as yellow oil.

**Retention factor:** 0.33 (DCM/MeOH 50:1 v/v). **¹H NMR:** (400 MHz, DMSO-d₆) *δ* (ppm) 8.12 (t, *J =* 1.7 Hz, 1H, *H*4), 7.47 (dd, *J* = 7.4, 1.7 Hz, 1H, H5), 7.45 - 7.41 (m, 1H, *H*8), 7.32 (td, *J* = 7.5, 1.8 Hz, 1H, *H*7), 7.27 (td, *J* = 7.3, 1.5 Hz, 1H, *H*6), 4.93 (d, *J* = 1.9 Hz, 2H, *H*2). **¹³C{¹H} NMR:** (101 MHz, DMSO-d₆) *δ* (ppm) 161.3 (*C*4), 131.9 (*C*7), 130.9 (*C*5), 130.1 (C8), 129.3 (C8a), 127.0 (*C*4a), 126.7 (*C*6), 42.1 (*C*2). **⁷⁷Se{¹H} NMR:** (76 MHz, DMSO-d₆) *δ* (ppm) -112.31. **HRMS (+EI):** [M]⁺: *m*/*z* calcd. for C₈H₇NSe⁺: 196.97382, found: 196.97403. **IR:** (ATR, FT) *ṽ* (cm⁻¹) 3055 (vw), 3015 (vw), 2937 (vw), 2901 (vw), 2826 (vw), 1668 (vw), 1628 (s), 1585 (m), 1560 (w), 1464 (w), 1435 (w), 1377 (w), 1290 (w), 1258 (m), 1202 (m), 1182 (m), 1159 (w), 1123 (w), 1072 (w), 1057 (m), 1030 (w), 982 (vw), 949 (m), 930 (w), 899 (m), 862 (w), 841 (m), 748 (vs), 714 (vs), 658 (w), 644 (w), 615 (w). **Elemental Anal.** Calcd. for C₈H₇NSe: C, 48.99; H, 3.60; N, 7.14. Found: C, 49.20; H, 3.98; N, 7.25.

### Example 3: 8-Methyl-2H-benzo[e][1,3]thiazine (1b-S)

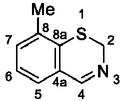

Compound **2b-S** was used as starting material. Compound **1b-S** was purified by flash chromatography using DCM/EtOAc (50:1 v/v) as eluent and obtained in 86% yield (422 mg, 2.59 mmol) as yellow oil.

**Retention factor:** 0.28 (DCM/EtOAc 50:1 v/v). **¹H NMR:** (300 MHz, DMSO-d₆) *δ* (ppm) 8.23 (t, *J* = 1.9 Hz, 1H, *H*4), 7.37 - 7.27 (m, 2H, *H*5,7), 7.17 (t, *J* = 7.5 Hz, 1H, *H*6), 4.77 (d, *J* = 1.9 Hz, 2H, *H*2), 2.23 (s, 3H, -C*H*₃). **¹³C{¹H} NMR:** (75 MHz, CDCl₃) *δ* (ppm) 160.5 (*C*4), 134.7 (*C*8), 133.8 (*C*8a), 133.1 (*C*5/7), 127.6 (*C*5/7), 126.5 (*C*4a), 125.0 (*C*6), 47.1 (*C*2), 18.8 (-CH₃). **HRMS (+EI):** [M]⁺: *m*/*z* calcd. for C₉H₉NS⁺: 163.04502, found: 163.04218. **IR:** (ATR, FT) *ṽ* (cm⁻¹) 3057 (vw), 3011 (vw), 2972 (vw), 2941 (w), 2889 (w), 2827 (vw), 1630 (s), 1582 (m), 1574 (m), 1452 (m), 1423 (m), 1377 (m), 1364 (m), 1294 (w), 1273 (w), 1256 (m), 1227 (m), 1202 (w), 1163 (w), 1117 (vw), 1061 (m), 1034 (m), 949 (s), 910 (w), 893 (w), 797 (s), 775 (vs), 737 (vs), 719 (vs), 690 (w). **Elemental Anal.** Calcd. for C₉H₉NS · 1/10H₂O: C, 65.50; H, 5.62; N, 8.49. Found: C, 65.31; H, 5.64; N, 8.65.

### Example 4: 8-Bromo-2H-benzo[e][1,3]thiazine (1c-S)

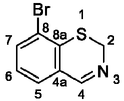

Compound **2c-S** was used as starting material. Compound **1c-S** was purified by flash chromatography using DCM/EtOAc (50:1 v/v) as eluent and obtained in 71% yield (487 mg, 2.14 mmol) as light orange solid.

**Retention factor:** 0.37 (DCM/EtOAc 50:1 v/v). **Melting point:** 40 °C. **¹H NMR:** (300 MHz, CDCl₃) *δ* (ppm) 8.13 (t, *J* = 1.9 Hz, 1H, *H*4), 7.55 (dd, *J* = 8.0, 1.3 Hz, 1H, *H*7), 7.27 (dd, *J* = 7.6, 1.4 Hz, 1H, *H*5), 7.10 (t, *J* = 7.8 Hz, 1H, *H*6), 4.83 (d, *J*= 1.9 Hz, 2H, *H*2). **¹³C{¹H} NMR:** (75 MHz, CDCl₃) *δ* (ppm) 160.7 (*C*4), 137.6 (*C*8a), 135.7 (*C*7), 129.0 (*C*4a), 128.8 (*C*5), 126.6 (*C*6), 122.1 (*C*8), 48.9 (*C*2). **HRMS (+EI):** [M]⁺: *m*/*z* calcd. for C₈H₆BrNS⁺: 226.93988, found: 226.94039. **IR:** (ATR, FT) *ṽ* (cm⁻¹) 3061 (vw), 3044 (vw), 2961 (vw), 2897 (vw), 2833 (vw), 1632 (w), 1616 (m), 1580 (w), 1543 (w), 1441 (w), 1414 (m), 1364 (m), 1288 (w), 1279 (w), 1221 (m), 1207 (w), 1182 (m), 1142 (m), 1105 (m), 1057 (m), 962 (w), 943 (s), 903 (w), 881 (w), 771 (s), 744 (vs), 725 (m), 710 (vs), 687 (m). **Elemental Anal.** Calcd. for C₈H₆BrNS: C, 42.12; H, 2.65; N, 6.14. Found: C, 42.25; H, 2.86; N, 6.12.

### Example 5: 8-Methoxy-2H-benzo[e][1,3]thiazine (1d-S)

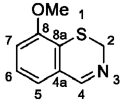

Compound **2d-S** was used as starting material. Compound **1d-S** was purified by flash chromatography using *n*-pentane/EtOAc (1:1 v/v) as eluent and obtained in 56% yield (300 mg, 1.67 mmol) as yellow oil.

**Retention factor:** 0.27 (DCM/EtOAc 20:1 v/v). **¹H NMR:** (300 MHz, DMSO-d₆) *δ* (ppm) 8.24 (t, *J =* 1.9 Hz, 1H, *H*4), 7.24 (dd, *J* = 8.4, 7.2 Hz, 1H. *H*6), 7.15 - 7.12 (m, 1H, *H*7), 7.12 - 7.09 (m, 1H, *H*5), 4.72 (d, *J* = 1.9 Hz, 2H, *H*2), 3.84 (s, 3H, -OC*H*₃). **¹³C{¹H} NMR:** (75 MHz, DMSO-d₆) *δ*(ppm) 160.1 (C4), 154.4 (*C*8), 127.2 (*C*4a), 125.9 (*C*6), 122.8 (C8a), 122.2 (C5), 114.0 (*C*7), 56.0 (-OCH₃), 46.5 (*C*2). **HRMS (+EI):** [M]⁺: *m*/*z* calcd. for C₉H₉NOS⁺: 179.03994, found: 179.04148. **IR:** (ATR, FT) *ṽ* (cm⁻¹) 3063 (vw), 3007 (vw), 2961 (vw), 2937 (w), 2887 (vw), 2835 (w), 1666 (w), 1628 (m), 1591 (w), 1564 (m), 1464 (s), 1433 (s), 1366 (m), 1315 (m), 1259 (vs), 1225 (m), 1204 (w), 1188 (w), 1173 (w), 1151 (vw), 1115 (vw), 1092 (m), 1053 (m), 970 (s), 947 (s), 905 (w), 879 (w), 789 (s), 775 (s), 733 (m), 716 (s), 698 (w), 625 (m). **Elemental Anal.** Calcd. for C₉H₉NOS · 2/10H₂O: C, 59.12; H, 5.18; N, 7.66. Found: C, 59.04; H, 4.99; N, 7.81.

### Example 6: Methyl 2H-benzo[e][1,3]thiazine-8-carboxylate (1e-S)

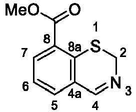

Compound **2e-S** was used as starting material. Compound **1e-S** was purified by flash chromatography using n-pentane/EtOAc (3:1 v/v) and obtained in 73% yield (456 mg, 2.20 mmol) as a slightly yellow solid.

**Retention factor:** 0.16 (*n*-pentane/EtOAc 1:1 v/v). **¹H NMR:** (400 MHz, DMSO-d₆) *δ* (ppm) 8.30 (t, *J* = 1.8 Hz, 1H, *H*4), 7.98 (dd, *J* = 7.9, 1.5 Hz, 1H, *H*7), 7.72 (dd, *J* = 7.5, 1.5 Hz, 1H, *H*5), 7.36 (t, *J* = 7.7 Hz, 1H, *H*6), 4.65 (d, *J* = 1.8 Hz, 2H, *H*2), 3.86 (s, 3H, - COOC*H*₃). **¹³C{¹H} NMR:** (100 MHz, DMSO-d₆) *δ* (ppm) 165.1 (-*C*OOCH₃), 159.9 (*C*4), 138.7 (*C*8a), 134.0 (*C*5), 133.6 (*C*7), 127.7 (*C*4a), 127.0 (*C*8), 124.8 (*C*6), 52.4 (*C*2), 46.9 (-COO*C*H₃). **HRMS (+APCI):** [M+H]⁺: *m*/*z* calcd. for C₁₀H₁₀NO₂S⁺: 208.0427, found: 208.0425. **IR:** (ATR, FT) *ṽ* (cm⁻¹) 3045 (vw), 2991 (vw), 2949 (w), 2914 (w), 2853 (vw), 1709 (s), 1670 (w), 1630 (m), 1582 (w), 1562 (m), 1450 (w), 1437 (m), 1420 (m), 1302 (w), 1271 (s), 1227 (m), 1209 (w), 1190 (s), 1140 (vs), 1117 (m), 1057 (m), 1011 (w), 989 (w), 960 (m), 945 (m), 922 (m), 887 (w), 816 (m), 758 (vs), 744 (vs), 714 (m), 687 (m).

### Example 7: 7-Methyl-2H-benzo[e][1,3]thiazine (1f) and 5-Methyl-2H-benzo[e][1,3]thiazine (1g)

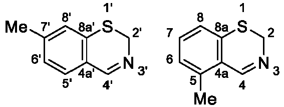

Compound **2f/g-S** was used as starting material. Compounds **1f-S** and **1g-S** were purified by flash chromatography using *n*-pentane/EtOAc (1:1 v/v) and obtained as 7.7:1 mixture **(1f-S/1g-S)** in 88% yield (429 mg, 2.63 mmol) as a yellow liquid.

**Retention factor:** 0.49 (n-pentane/EtOAc 1:1 v/v). **¹H NMR:** (400 MHz, DMSO-d₆) *δ* (ppm) 8.46 (t, *J =* 1.8 Hz, 1H, *H*4), 8.19 (t, *J* = 1.8 Hz, 1H, *H*4'), 7.35 (d, *J* = 7.7 Hz, 1H, *H*5'), 7.26 (t, *J* = 7.7 Hz, 1H, *H*7), 7.15 (d, *J* = 7.8 Hz, 1H, *H*6/8), 7.10 (s, 1H, *H*8'), 7.07 (d, *J* = 7.7 Hz, 1H, *H*6'), 4.75 (d, *J* = 1.9 Hz, 2H, *H*2'), 4.64 (d, *J* = 1.8 Hz, 2H, *H*2), 2.43 (s, 3H, -C*H*₃), 2.29 (s, 3H, -C*H*₃'). **¹³C{¹H} NMR:** (100 MHz, DMSO-d₆) *δ* (ppm) 159.8 (*C*4'), 157.9 (*C*4), 142.2 (*C*7'), 138.2 (*C*5), 135.0 (*C*8a), 133.9 (*C*8a'), 131.2 (*C*7), 130.0 (*C*5'), 128.1 (C6/8), 127.4 (*C*8'), 126.8 (*C*6'), 125.4 (*C*4a), 125.0 (*C*6/8), 124.4 (*C*4a'), 47.1 (*C*2'), 46.4 (*C*2), 21.0 (-*C*H₃'), 18.3 (-*C*H₃). **HRMS (+APCI):** [M+H]⁺: *m*/*z* calcd. for C₉H_{1O}NS⁺:, 164.0528 found: 164.0550. **IR:** (ATR, FT) *ṽ* (cm⁻¹) 3051 (vw), 3015 (w), 2943 (w), 2918 (w), 2887 (w), 2829 (w), 1722 (vw), 1622 (s), 1597 (vs), 1553 (m), 1481 (w), 1450 (m), 1425 (m), 1400 (w), 1364 (w), 1294 (w), 1265 (m), 1213 (s), 1196 (m), 1175 (w), 1142 (w), 1070 (m), 1036 (w), 947 (s), 924 (s), 891 (m), 876 (m), 812 (vs), 777 (m), 733 (s), 706 (m), 690 (w), 644 (w). **Elemental Anal.** Calcd. for C₉H₉NS . 1/10 H₂O: C, 65.50; H, 5.62; N, 8.49. Found: C, 65.69; H, 5.64; N, 8.67.

### Example 8: 7-Bromo-2H-benzo[e][1,3]thiazine (1h-S) and 5-Bromo-2H- benzo[e][1,3]thiazine (1i-S)

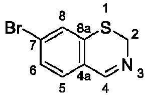

Compound **2h/i-S** was used as starting material. Compound **1h-S** was purified from the mixture of compounds **1h-S** and **1i-S** by flash chromatography using n-pentane/EtOAc (1:1 v/v) as eluent and obtained in 48% yield (326 mg, 1.43 mmol) as light orange solid. **Retention factor:** 0.24 (n-pentane/EtOAc 4:1 v/v). **Melting point:** 57 °C. **¹H NMR:** (300 MHz, DMSO-d₆) *δ* (ppm) 8.26 (t, *J* = 2.0 Hz, 1H, *H*4), 7.56 (d, *J* = 1.8 Hz, 1H, *H*8), 7.47 (dd, *J* = 8.1, 1.8 Hz, 1H, *H*6), 7.42 (d, *J =* 8.1 Hz, 1H, H5), 4.80 (d, *J =* 1.9 Hz, 2H, *H*2). **¹³C{¹H} NMR:** (75 MHz, DMSO-d₆) *δ* (ppm) 159.3 (*C*4), 136.8 (*C*4a), 131.7 (*C*5), 129.4 (C8), 129.0 (C7), 125.5 (C8a), 125.0 (*C*7), 46.8 (C2). **HRMS (+EI):** [M]⁺: *m*/*z* calcd. for C₈H₆BrNS⁺: 226.93988, found: 226.93876. **IR:** (ATR, FT) *ṽ* (cm⁻¹) 2966 (vw), 2924 (vw), 2893 (vw), 2853 (vw), 2827 (vw), 1724 (vw), 1678 (vw), 1630 (m), 1620 (m), 1574 (m), 1545 (m), 1462 (w), 1431 (w), 1421 (w), 1391 (w), 1354 (w), 1288 (w), 1281 (w), 1250 (w), 1217 (w), 1194 (w), 1138 (w), 1086 (w), 1063 (w), 966 (w), 943 (m), 924 (s), 858 (s), 804 (vs), 766 (m), 727 (m), 700 (m), 673 (w), 608 (vw). **Elemental Anal.** Calcd. for C₈H₆BrNS: C, 42.12; H, 2.65; N, 6.14. Found: C, 42.26; H, 2.95; N, 6.18.

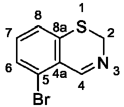

Compound **2h/i-S** was used as starting material. Compound **1i-S** was purified from the mixture of compounds **1h-S** and **1i-S** by flash chromatography using n-pentane/EtOAc (1:1 v/v) as eluent and obtained in 44% yield (301 mg, 1.32 mmol) as yellow solid. **Retention factor:** 0.64 (n-pentane/EtOAc 4:1 v/v). **Melting point:** 67 °C. **¹H NMR:** (300 MHz, DMSO-d₆) *δ* (ppm) 8.40 (d, *J* = 1.8 Hz, 1H, *H*4), 7.54 (dd, *J* = 7.8, 1.3 Hz, 1H, *H*6), 7.40 (d, *J* = 7.5 Hz, 1H, *H*8), 7.31 (t, *J* = 7.8 Hz, 1H, *H*7), 4.75 (d, *J* = 1.8 Hz, 2H, *H*2). **¹³C{¹H} NMR:** (75 MHz, DMSO-d₆) *δ* (ppm) 158.2 (*C*4), 138.5 (*C*8a), 132.8 (*C*7), 130.3 (*C*6), 127.0 (*C*8), 124.9 (*C*4a/5), 124.8 (*C*4a/5), 46.8 (C2). **HRMS (+EI):** [M]⁺: *m*/*z* calcd. for C₈H₆BrNS⁺: 226.93988, found: 226.94203. **IR:** (ATR, FT) *ṽ* (cm⁻¹) 1618 (m), 1572 (m), 1543 (m), 1429 (s), 1366 (w), 1265 (w), 1213 (m), 1190 (m), 1086 (m), 1022 (w), 986 (vw), 949 (m), 914 (m), 891 (w), 874 (w), 849 (m), 822 (w), 775 (vs), 756 (s), 735 (vs), 717 (s), 683 (s), 646 (w), 633 (w), 611 (w). **Elemental Anal.** Calcd. for C₈H₆BrNS: C, 42.12; H, 2.65; N, 6.14. Found: C, 42.00; H, 2.78; N, 5.99.

### Example 9: 7-Fluoro-2H-benzo[e][1,3]thiazine (1j-S) and 5-Fluoro-2H-benzo[e][1,3]thiazine (1k-S)

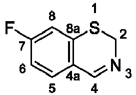

Compound **2j/k-S** was used as starting material. Compound **1j-S** was purified from the mixture of compounds **1j-S** and **1k-S** by flash chromatography using *n*-pentane/EtOAc (2:1 v/v) and obtained in 78% yield (393 mg, 2.35 mmol) as a slightly yellow oil.

**Retention factor:** 0.24 (*n*-pentane/EtOAc 2:1 v/v). **¹H NMR:** (400 MHz, DMSO-d₆) *δ* (ppm) 8.24 (t, *J* = 1.7 Hz, 1H, *H*4), 7.56 (dd, *J* = 8.5, 6.0 Hz, 1H, *H*5), 7.23 (dd, *J* = 9.2, 2.5 Hz, 1H, *H*8), 7.10 (td, *J* = 8.7, 2.6 Hz, 1H, *H*6), 4.80 (d, *J =* 1.9 Hz, 2H, *H*2). **¹³C{¹H} NMR:** (100 MHz, DMSO-d₆) *δ* (ppm) 163.2 (d, *J* = 251.8 Hz, *C*7), 159.0 (*C*4), 137.2 (d, *J* = 9.9 Hz, *C*8a), 132.6 (d, *J* = 9.8 Hz, *C*5), 123.5 (d, *J* = 3.0 Hz, *C*4a), 114.1 (d, *J* = 24.4 Hz, *C*8), 113.2 (d, *J* = 22.0 Hz, *C*6), 46.8 (*C*2). **¹⁹F{¹H} NMR:** (283 MHz, DMSO-d₆) *δ* (ppm) -106.70 (*F*7). **HRMS (+APCI):** [M+H]⁺: *m*/*z* calcd. for C₈H₇FNS⁺: 168.0278, found: 168.0284. **IR:** (ATR, FT) *ṽ* (cm⁻¹) 2945 (vw), 2891 (vw), 2829 (vw), 1626 (s), 1595 (s), 1566 (vs), 1479 (s), 1427 (w), 1404 (w), 1364 (w), 1300 (w), 1259 (s), 1221 (vs), 1205 (s), 1123 (vw), 1103 (vw), 1057 (s), 943 (s), 926 (s), 905 (m), 858 (s), 808 (s), 731 (s), 702 (m), 689 (m), 644 (s). **Elemental Anal.** Calcd. for C₈H₆FNS: C, 57.47; H, 3.62; N, 8.38. Found: C, 57.22; H, 3.77; N, 8.50.

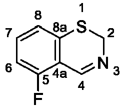

Compound **2j/k-S** was used as starting material. Compound **1k-S** was purified from the mixture of compounds **1j-S** and **1k-S** by flash chromatography using n-pentane/EtOAc (2:1 and 8:1 v/v) as eluent and obtained in 7% yield (35 mg, 0.21 mmol) as slightly yellow oil.

**Retention factor:** 0.31 (n-pentane/EtOAc 8:1 v/v). **IR:** (ATR, FT) *ṽ* (cm⁻¹) 2962 (vw), 2891 (vw), 2829 (vw), 1626 (s), 1601 (s), 1564 (s), 1450 (s), 1427 (w), 1373 (m), 1294 (w), 1271 (vw), 1242 (s), 1217 (m), 1198 (w), 1165 (m), 1150 (w), 962 (m), 951 (m), 928 (m), 897 (s), 843 (m), 777 (vs), 739 (m), 717 (m), 687 (m).

### Example 10: 7-Methoxy-2H-benzo[e][1,3]thiazine (1l-S)

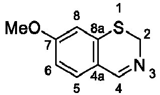

Compound **2l/m-S** was used as starting material. Compound **1l-S** was purified by flash chromatography using n-pentane/EtOAc (1:1 and 2:1 v/v) as eluent and obtained in 72% yield (384 mg, 2.14 mmol) as slightly yellow solid.

**Retention factor:** 0.27 (n-pentane/EtOAc 2:1 v/v). **Melting point:** 42 °C. **¹H NMR:** (300 MHz, DMSO-d₆) *δ* (ppm) 8.14 (t, *J* = 1.9 Hz, 1H, *H*4), 7.41 (d, *J* = 8.3 Hz, 1H, *H*5), 6.85 (d, *J* = 2.5 Hz, 1H, *H*8), 6.81 (dd, *J* = 8.4, 2.5 Hz, 1H, *H*6), 4.74 (d, *J* = 1.8 Hz, 2H, *H*2), 3.79 (s, 3H, -OC*H*₃). **¹³C{¹H} NMR:** (75 MHz, DMSO-d₆) *δ* (ppm) 161.5 (*C*7), 159.4 (*C*4), 136.1 (*C*8a), 131.9 (*C*5), 120.3 (*C*4a), 112.3 (*C*6), 111.9 (*C*7), 55.6 (-O*C*H₃), 47.1 (*C*2). **HRMS (+EI):** [M]⁺: *m*/*z* calcd. for C₉H₉NOS⁺: 179.03994, found: 179.03986. **IR:** (ATR, FT) *ṽ* (cm⁻¹) 3011 (vw), 2999 (w), 2974 (vw), 2943 (w), 2901 (vw), 2837 (vw), 1618 (m), 1591 (s), 1551 (s), 1487 (m), 1464 (m), 1454 (m), 1427 (w), 1408 (w), 1367 (w), 1306 (w), 1277 (s), 1232 (vs), 1217 (s), 1178 (m), 1061 (s), 1028 (s), 972 (m), 947 (m), 924 (m), 885 (m), 872 (m), 804 (s), 731 (m), 704 (m), 689 (m), 648 (s), 608 (w). **Elemental Anal.** Calcd. for C₉H₉NOS: C, 60.31; H, 5.06; N, 7.81. Found: C, 60.42; H, 5.15; N, 7.73.

### Example 11: 6-Methyl-2H-benzo[e][1,3]thiazine (1n-S)

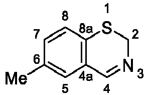

Reaction conditions based on 3.20 mmol 4-methylbenzenethiol **(2n-S)** were used. Compound 1n-S was purified by flash chromatography using DCM/EtOAc (50:1 v/v) as eluent and obtained in 81% yield (424 mg, 2.60 mmol) as slightly yellow solid. **Retention factor:** 0.32 (DCM/EtOAc 50:1 v/v). **Melting point:** 40 °C. **¹H NMR:** (300 MHz, DMSO-d₆) *δ* (ppm) 8.20 (t, *J* = 1.9 Hz, 1H, *H*4), 7.30 (d, *J* = 1.9 Hz, 1H, *H*5), 7.21 (dd, *J* = 8.0, 1.8 Hz, 1H, *H*7), 7.17 (d, *J* = 7.9 Hz, 1H, *H*8), 4.74 (d, *J* = 1.8 Hz, 2H, *H*2), 2.28 (s, 3H, -C*H*₃). **¹³C{¹H} NMR:** (75 MHz, DMSO-d₆) *δ*(ppm) 160.1 (*C*4), 135.5 (*C*6), 132.6 (*C*7), 130.5 (*C*8a), 130.4 (*C*5), 127.0 (*C*8), 126.6 (*C*4a), 47.2 (*C*2), 20.3 (-*C*H₃). **HRMS (+EI):** [M]⁺: *m*/*z* calcd. for C₉H₉NS⁺: 163.04502, found: 163.04375. **IR:** (ATR, FT) *ṽ* (cm⁻¹) 3047 (vw), 3030 (vw), 3011 (vw), 2974 (vw), 2945 (vw), 2916 (vw), 2887 (w), 2839 (vw), 2831 (vw), 1628 (m), 1618 (m), 1601 (w), 1556 (w), 1470 (w), 1456 (w), 1431 (w), 1400 (vw), 1379 (w), 1360 (w), 1296 (vw), 1277 (vw), 1263 (vw), 1234 (w), 1196 (w), 1161 (w), 1109 (vw), 1072 (w), 1041 (w), 1009 (vw), 949 (m), 905 (w), 885 (w), 812 (vs), 775 (w), 754 (w), 733 (m), 708 (m), 685 (w), 629 (w). **Elemental Anal.** Calcd. for C₉H₉NS: C, 66.22; H, 5.56; N, 8.58. Found: C, 66.12; H, 5.54; N, 8.56.

### Example 12: 6-Bromo-2H-benzo[e][1,3]thiazine (1o-S)

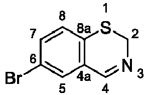

Compound **2o-S** was used as starting material. Compound **1o-S** was purified by flash chromatography using *n*-pentane/EtOAc (4:1 v/v) as eluent and obtained in 66% yield (450 mg, 1.97 mmol) as slightly yellow solid.

**Retention factor:** 0.4 (*n*-pentane/EtOAc 4:1 v/v). **Melting point:** 62 °C. **¹H NMR:** (300 MHz, DMSO-d₆) *δ* (ppm) 8.26 (t, *J* = 1.9 Hz, 1H, H4), 7.75 (d, *J* = 2.2 Hz, 1H, *H*5), 7.57 (dd, *J* = 8.3, 2.2 Hz, 1H, *H*7), 7.27 (d, *J* = 8.3 Hz, 1H, *H*8), 4.82 (d, *J* = 1.9 Hz, 2H, *H*2). **¹³C{¹H} NMR:** (75 MHz, DMSO-d₆) *δ* (ppm) 158.9 (C4), 134.4 (C7), 133.6 (C8a), 132.4 (C5), 129.1 (C8), 128.1 (C4a), 118.1 (C6), 46.9 (C2). **HRMS (+EI):** [M]⁺: *m*/*z* calcd. for C₈H₆BrNS⁺: 226.93988, found: 226.94151. **IR:** (ATR, FT) *ṽ* (cm⁻¹) 3032 (w), 2995 (vw), 2959 (vw), 2935 (vw), 2910 (vw), 1624 (m), 1572 (w), 1543 (w), 1458 (m), 1421 (w), 1393 (w), 1352 (w), 1279 (w), 1256 (w), 1207 (w), 1186 (m), 1151 (w), 1080 (s), 947 (m), 926 (s), 872 (m), 833 (vs), 762 (vs), 719 (s), 677 (w), 650 (w). **Elemental Anal.** Calcd. for C₈H₆BrNS: C, 42.12; H, 2.65; N, 6.14. Found: C, 42.15; H, 2.88; N, 6.22.

### Example 13: 6-Fluoro-2H-benzo[e][1,3]thiazine (1p-S)

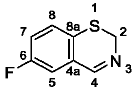

Compound **2p-S** was used as starting material. Compound **1p-S** was purified by flash chromatography using n-pentane/EtOAc (2:1 v/v) as eluent and obtained in 41% yield (208 mg, 1.25 mmol) as yellow oil.

**Retention factor:** 0.53 (n-pentane/EtOAc 2:1 v/v). **¹H NMR:** (300 MHz, DMSO-d₆) *δ* (ppm) 8.26 (t, *J* = 1.9 Hz, 1H, *H*4), 7.44 (dd, *J* = 8.9, 2.8 Hz, 1H, *H*5), 7.36 (dd, *J* = 8.6, 5.3 Hz, 1H, *H*8), 7.28 (td, *J* = 8.6, 2.8 Hz, 1H, *H*7), 4.79 (d, *J* = 1.8 Hz, 2H, *H*2). **¹³C{¹H} NMR:** (75 MHz, DMSO-d₆) *δ* (ppm) 160.2 (d, *J* = 243.4 Hz, C6), 159.3 (d, *J* = 1.9 Hz, C4), 129.5 (d, *J* = 2.9 Hz, C8a), 129.0 (d, *J* = 7.2 Hz, C8), 127.8 (d, *J* = 7.0 Hz, C4a), 119.1 (d, *J* = 22.4 Hz, C7), 116.5 (d, *J* = 22.7 Hz, C5), 47.2 (C2). **¹⁹F{¹H} NMR:** (283 MHz, DMSO-d₆) *δ* (ppm) -115.76. **HRMS (+EI):** [M]⁺: *m*/*z* calcd. for C₈H₆FNS⁺: 167.01995, found: 167.02095. **IR:** (ATR, FT) *ṽ* (cm⁻¹) 3055 (vw), 3011 (vw), 2945 (vw), 2891 (vw), 2827 (vw), 1672 (vw), 1628 (m), 1601 (w), 1570 (m), 1470 (s), 1427 (w), 1410 (w), 1360 (w), 1296 (m), 1256 (s), 1234 (s), 1202 (m), 1142 (s), 1123 (w), 1076 (w), 959 (m), 906 (m), 864 (m), 814 (vs), 729 (m), 704 (w), 687 (vw), 629 (s). **Elemental Anal.** Calcd. for C₈H₆FNS: C, 57.47; H, 3.62; N, 8.38. Found: C, 56.97; H, 4.02; N, 8.59.

### Example 14: 6-Methoxy-2H-benzo[e][1,3]thiazine (1q-S)

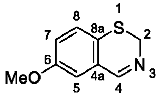

Compound **2q-S** was used as starting material. Compound **1q-S** was purified by flash chromatography using n-pentane/EtOAc (2:1 v/v) and obtained in 49% yield (264 mg, 1.65 mmol) as a yellow oil.

**Retention factor:** 0.31 (*n*-pentane/EtOAc 2:1 v/v). **¹H NMR:** (400 MHz, DMSO-d₆) *δ* (ppm) 8.24 (t, *J =* 1.7 Hz, 1H), 7.21 (d, *J =* 8.5 Hz, 1H, *H*8), 7.15 (d, *J* = 2.8 Hz, 1H, *H*5), 7.01 (dd, *J* = 8.5, 2.8 Hz, 1H, *H*7), 4.73 (d, *J* = 1.9 Hz, 2H, *H*2), 3.77 (s, 3H, -OC*H*₃). **¹³C{¹H} NMR:** (100 MHz, DMSO-d₆) *δ* (ppm) 160.1 (*C*4), 157.7 (*C*6), 128.1 (*C*8), 127.5 (*C*4a), 124.3 (*C*8a), 118.3 (*C*7), 115.0 (*C*5), 55.4 (-O*C*H₃), 47.4 (*C*2). **HRMS (+APCI):** [M+H]⁺: *m*/*z* calcd. for C₉H₁₀NOS⁺: 180.0478, found: 180.0477. **IR:** (ATR, FT) *ṽ* (cm⁻¹) 3003 (vw), 2955 (w), 2935 (w), 2893 (w), 2833 (w), 1720 (vw), 1680 (vw), 1626 (m), 1595 (m), 1562 (s), 1477 (s), 1464 (s), 1441 (m), 1425 (w), 1414 (w), 1360 (w), 1315 (m), 1271 (vs), 1240 (vs), 1202 (m), 1188 (m), 1161 (s), 1078 (w), 1030 (s), 949 (m), 937 (m), 901 (m), 864 (w), 849 (m), 812 (vs), 729 (s), 706 (w), 689 (w), 631 (s), 604 (vw). **Elemental Anal.** Calcd. for C₉H₉NOS · 0.1 H₂O: C, 59.71; H, 5.12; N, 7.74. Found: C, 59.61; H, 5.18; N, 7.65.

### Example 15: 6-tert-Butyl-2H-benzo[e][1,3]thiazine (1r-S)

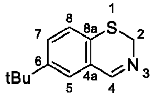

Compound **2r-S** was used as starting material. Compound **1r-S** was purified by flash chromatography using n-pentane/EtOAc (3:1 v/v) and obtained in 90% yield (554 mg, 2.70 mmol) as an orange oil.

**Retention factor:** 0.5 (*n*-pentane/EtOAc 3:1 v/v). **¹H NMR:** (300 MHz, DMSO-d₆) *δ* (ppm) 8.27 (d, *J* = 1.9 Hz, 1H, *H*4), 7.53 (d, *J* = 2.2 Hz, 1H, *H*5), 7.43 (dd, *J* = 8.2, 2.3 Hz, 1H, *H*7), 7.21 (d, *J* = 8.1 Hz, 1H, *H*8), 4.75 (d, *J* = 1.9 Hz, 2H, *H*2), 1.27 (s, 9H, -C(C*H*₃)₃). **¹³C{¹H} NMR:** (75 MHz, DMSO-d₆) *δ* 160.5 (*C*4), 148.9 (*C*6), 130.6 (*C*8a), 129.0 (*C*7), 127.0 (C8), 126.8 (C5), 126.4 (C4a), 47.1 (C2), 34.2, 30.8 (-C(*C*H₃)₃). **HRMS (+APCI):** [M+H]⁺: m/z calcd. for C₁₂H₁₆NS⁺: 206.0998, found: 206.1012. **IR:** (ATR, FT) *ṽ* (cm⁻¹) 2961 (s), 2903 (w), 2868 (w), 1722 (w), 1628 (vs), 1553 (m), 1477 (s), 1464 (m), 1425 (w), 1394 (w), 1366 (m), 1296 (vw), 1277 (w), 1261 (m), 1205 (s), 1190 (s), 1161 (w), 1117 (m), 1068 (m), 1045 (vw), 1026 (vw), 949 (s), 935 (s), 899 (w), 879 (w), 829 (vs), 773 (s), 731 (s), 712 (w), 690 (w), 621 (m). **Elemental Anal.** Calcd. for C₁₂H₁₅NS · 1/5 H₂O: C, 68.99; H, 7.43; N, 6.70. Found: C, 69.10; H, 7.33; N, 6.83.

### Example 16: 6-(Trifluoromethyl)-2H-benzo[e][1,3]thiazine (1s-S)

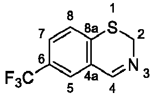

Compound **2s-S** was used as starting material. Compound **1s-S** was purified by flash chromatography using DCM/EtOAc (120:1 v/v) as eluent and obtained in 36% yield (237 mg, 1.09 mmol) as light-yellow solid.

**Retention factor:** 0.42 (DCM/EtOAc 120:1 v/v). **Melting point:** 58 °C. **¹H NMR:** (700 MHz, DMSO-d₆) *δ* (ppm) *δ* 8.37 (t, *J* = 2.0 Hz, 1H, *H*4), 7.91 (d, *J* = 2.2 Hz, 1H, *H*5), 7.70 (dd, *J* = 8.2, 2.1 Hz, 1H, *H*7), 7.52 (d, *J* = 8.2 Hz, 1H, *H*8), 4.89 (d, *J* = 1.9 Hz, 2H, *H*2) **¹³C{¹H} NMR:** (176 MHz, DMSO-d₆) *δ* (ppm) 159.2 (C4), 140.1 (C8a), 128.1 (C8), 128.0 (q, *J* = 3.6 Hz, *C*7), 126.71 (*C*4a), 126.67 (q, *J* = 4.0 Hz, *C*5), 126.4 (q, *J* = 32.4 Hz, *C*6), 123.8 (q, *J* = 272.0 Hz, -CF₃), 46.8 (*C*2 **¹⁹F{¹H} NMR:** (283 MHz, DMSO-d₆) *δ* (ppm) - 61.20 (-C*F*₃). **HRMS (+EI):** [M]⁺: *m*/*z* calcd. for C₉H₆F₃NS⁺: 217.01676, found: 217.01519. **IR:** (ATR, FT) *ṽ* (cm⁻¹) 3059 (vw), 3026 (vw), 2961 (vw), 2934 (vw), 2914 (vw), 2851 (vw), 2827 (vw), 1634 (m), 1610 (w), 1566 (w), 1416 (w), 1369 (vw), 1331 (s), 1302 (m), 1259 (m), 1207 (vw), 1186 (m), 1171 (m), 1142 (m), 1115 (vs), 1084 (vs), 989 (w), 951 (m), 932 (m), 899 (w), 841 (m), 795 (m), 719 (m), 700 (w), 636 (w). **Elemental Anal**. Calcd. for C₉H₆F₃NS: C, 49.77; H, 2.78; N, 6.45. Found: C, 50.02; H, 3.05; N, 6.46.

### Example 17: 6-(Methylsulfanyl)-2H-benzo[e][1,3]thiazine (1t-S)

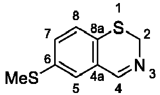

Compound **2t-S** was used as starting material. Compound **1t-S** was purified by flash chromatography using n-pentane/EtOAc (2:1 v/v) as eluent and obtained in 40% yield (235 mg, 1.20 mmol) as yellow solid.

**Retention factor:** 0.43 (n-pentane/EtOAc 2:1 v/v). **Melting point:** 42 °C. **¹H NMR:** (300 MHz, CDCl₃) *δ* (ppm) 8.17 (t, *J* = 1.9 Hz, 1H, *H*4), 7.22 (dd, *J* = 8.0, 2.1 Hz, 1H, *H*7), 7.20 (d, *J* = 1.7 Hz, 1H, *H*5), 7.16 (d, *J* = 8.0 Hz, 1H, *H*8), 4.75 (d, *J* = 1.9 Hz, 2H, *H*2), 2.48 (s, 3H, -SC*H*₃). **¹³C{¹H} NMR:** (75 MHz, CDCl₃) *δ* (ppm) 160.5 (C4), 136.5 (C6), 131.7 (C8a), 130.6 (C7), 128.3 (C5,8), 128.0 (C5,8), 127.4 (C4a), 48.0 (C2), 16.4 (-S*C*H₃). **HRMS (+EI):** [M]⁺: *m*/*z* calcd. for C₉H₉NS₂⁺: 195.01709, found: 195.01646. IR: (ATR, FT) *ṽ* (cm-¹) 2982 (vw), 2959 (vw), 2918 (w), 2887 (w), 2853 (vw), 2829 (vw), 1720 (vw), 1676 (m), 1626 (s), 1580 (w), 1543 (m), 1464 (m), 1427 (m), 1393 (w), 1356 (m), 1315 (w), 1281 (m), 1261 (w), 1234 (w), 1209 (m), 1188 (s), 1150 (w), 1107 (s), 1063 (m), 949 (s), 930 (s), 879 (w), 812 (s), 783 (vs), 750 (w), 725 (s), 681 (m). **Elemental Anal.** Calcd. for C₉H₉NS₂: C, 55.35; H, 4.65; N, 7.17. Found: C, 55.20; H, 4.75; N, 7.03.

### Example 18: 6,7-Dimethoxy-2H-benzo[e][1,3]thiazine (1u-S)

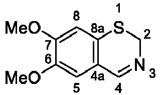

Compound **2u-S** was used as starting material. Compound **1u-S** was purified by flash chromatography using n-pentane/EtOAc (1:1 v/v) as eluent and obtained in 78% yield (492 mg, 2.35 mmol) as light yellow solid.

**Retention factor:** 0.2 (n-pentane/EtOAc 1:1 v/v). **Melting point:** 100 °C. **¹H NMR:** (300 MHz, DMSO-d₆) *δ* (ppm) 8.12 (t, *J* = 1.80 Hz, 1H, *H*4), 7.16 (s, 1H, *H*5), 6.88 (s, 1H, *H*8), 4.69 (d, *J* = 1.8 Hz, 2H, *H*2), 3.80 (s, 3H, C7-OC*H*₃), 3.76 (s, 3H, C6-O*C*H₃). **¹³C{¹H} NMR:** (75 MHz, DMSO-d₆) *δ* (ppm) 159.5 (*C*4), 151.7 (C7-O*C*H₃), 147.0 (C6-O*C*H₃), 126.4 (*C*8a), 119.9 (*C*4a), 113.4 (*C*5), 110.0 (*C*8), 55.9 (-O*C*H₃), 55.7 (-O*C*H₃), 47.3, (*C*2). **HRMS (+EI):** [M]⁺: *m*/*z* calcd. for C₁₀H₁₁NO₂S⁺: 209.05050, found: 209.05057. **IR:** (ATR, FT) *ṽ* (cm⁻¹) 3007 (vw), 2961 (vw), 2932 (vw), 2907 (vw), 2839 (vw), 1618 (m), 1593 (m), 1558 (m), 1504 (m), 1462 (w), 1441 (m), 1394 (w), 1352 (m), 1333 (m), 1263 (vs), 1225 (m), 1204 (m), 1167 (s), 1061 (vs), 1028 (m), 982 (m), 945 (m), 916 (m), 862 (m), 841 (m), 829 (m), 798 (s), 729 (w), 689 (w), 656 (vw), 611 (vw). **Elemental Anal.** Calcd. for C₁₀H₁₁NO₂S: C, 57.40; H, 5.30; N, 6.69. Found: C, 57.18; H, 5.46; N, 6.49.

### Example 19: 2H-naphtho[2,1-e][1,3]thiazine (1v-S)

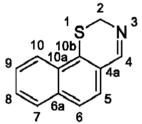

Compound **2v-S** was used as starting material. Compound **1v-S** was purified by flash chromatography using n-pentane/EtOAc (4:1 v/v) as eluent and obtained in 85% yield (509 mg, 2.56 mmol) as yellow solid.

**Retention factor:** 0.27 (*n*-pentane/EtOAc 4:1 v/v). **Melting Point:** 58 °C. **¹H NMR:** (300 MHz, DMSO-d₆) *δ* (ppm) 8.42 (t, *J* = 1.8 Hz, 1H, *H*4), 8.16-8.06 (m, 1H), 8.04-7.93 (m, 1H), 7.82 (d, *J* = 8.4 Hz, 1H, *H*6), 7.73 - 7.60 (m, 2H), 7.61 (d, *J* = 8.4 Hz, 1H, *H*5), 4.91 (d, *J* = 1.7 Hz, 2H, *H*2). **¹³C{¹H} NMR:** (75 MHz, DMSO-d₆) *δ* (ppm) 160.8 (*C*4), 134.3 (*C*6a), 133.8 (*C*10b), 129.4 (*C*10a), 128.6, 128.4, 127.1, 126.3 (*C*5), 125.3 (*C*6), 124.3, 124.2 (C4a), 47.2 (C2). **HRMS (+EI):** [M]⁺: *m*/*z* calcd. for C₁₂H₉NS⁺: 199.04502, found: 199.04534. **IR:** (ATR, FT) *ṽ* (cm⁻¹) 3051 (vw), 3030 (vw), 2963 (vw), 2934 (vw), 2880 (w), 2816 (vw), 1630 (m), 1609 (m), 1589 (w), 1547 (w), 1502 (w), 1454 (w), 1420 (w), 1373 (vw), 1313 (w), 1273 (vw), 1258 (w), 1211 (w), 1196 (w), 1157 (w), 1142 (w), 1038 (m), 1022 (w), 972 (w), 943 (m), 905 (w), 862 (m), 829 (w), 806 (vs), 775 (s), 743 (vs), 719 (m), 671 (w), 646 (m), 625 (w). **Elemental Anal.** Calcd. for C₁₂H₉NS: C, 72.33; H, 4.55; N, 7.03. Found: C, 72.05; H, 4.51; N, 6.98.

### Example 20: 3H-Naphtho[1,2-e][1,3]thiazine (1w-S)

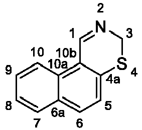

Compound **2w-S** was used as starting material. Compound **1w-S** was purified by flash chromatography using n-pentane/EtOAc (4:1 v/v) as eluent and obtained in 82% yield (489 mg, 2.45 mmol) as yellow solid.

**Retention factor:** 0.32 (n-pentane/EtOAc 4:1 v/v). **Melting Point:** 77 °C. **¹H NMR:** (300 MHz, DMSO-d₆) *δ* (ppm) 8.99 (t, *J* = 1.8 Hz, 1H, *H*1), 8.29 (d, *J* = 8.4 Hz, 1H, *H*10), 7.97 (d, *J* = 8.6 Hz, 1H, *H*6), 7.95 (d, *J* = 7.7 Hz, 1H, *H*7), 7.63 (ddd, *J* = 8.5, 6.9, 1.5 Hz, 1H, *H*9), 7.54 (ddd, *J* = 8.0, 6.9, 1.2 Hz, 1H, *H*8), 7.47 (d, *J* = 8.6 Hz, 1H, *H*5), 4.74 (d, *J* = 1.7 Hz, 2H, *H*3). **¹³C{¹H} NMR:** (75 MHz, DMSO-d₆) *δ* (ppm) 157.3 (C1), 136.1 (C4a), 131.7 (C6), 131.4 (C6a), 130.8 (C10a), 128.9 (C7), 128.3 (C9), 125.8 (C8), 125.0 (C5), 122.6 (C10b), 121.6 (C10), 47.0 (C3). **HRMS (+EI):** [M]⁺: *m*/*z* calcd. for C₁₂H₉NS⁺: 199.04502, found: 199.04457. **IR:** (ATR, FT) *ṽ* (cm⁻¹) 3063 (vw), 3045 (vw), 3032 (vw), 2970 (vw), 2893 (vw), 2837 (vw), 1620 (w), 1609 (m), 1585 (w), 1551 (w), 1506 (w), 1487 (vw), 1452 (vw), 1427 (vw), 1396 (w), 1371 (vw), 1319 (vw), 1259 (vw), 1204 (w), 1182 (m), 1167 (w), 1134 (m), 1053 (m), 1020 (vw), 964 (w), 933 (m), 879 (m), 856 (w), 808 (vs), 771 (s), 739 (s), 721 (m), 669 (w), 648 (w), 619 (w). **Elemental Anal.** Calcd. for C₁₂H₉NS: C, 72.33; H, 4.55; N, 7.03. Found: C, 72.05; H, 4.55; N, 6.88.

### Example 21: 2H,8H-benzo[1,2-e:5,4-e']bis([1,3]thiazine (1x-S)

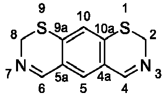

Compound **2x-S** was used as starting material. Compound **1x-S** was synthesised using 10 mL TFA instead of 5 mL and purified by flash chromatography using EtOAc/methanol (20:1 v/v) and obtained in 51% yield (335 mg, 1.52 mmol) as yellow solid.

**Retention factor:** 0.23 (EtOAc/methanol 20:1 v/v). **Melting point:** 146 °C. **¹H NMR:** (300 MHz, DMSO-d₆) *δ* (ppm) 8.21 (t, *J* = 1.9 Hz, 2H, *H*4,6), 7.55 (s, 1H, *H*5), 7.25 (s, 1H, *H*10), 4.84 (d, *J =* 1.9 Hz, 4H, *H*2,8). **¹³C{¹H} NMR:** (75 MHz, DMSO-d₆) *δ* (ppm) 159.1 (C4,6), 139.1 (*C*9a,10a), 131.0 (*C*5), 125.0 (*C*10), 123.9 (*C*4a,5a), 47.0 (*C*2,8). **HRMS (+APCI):** [M+H]⁺: *m*/*z* calcd. for C₁₀H₉N₂S₂⁺: 221.0202, found: 221.0194. **IR:** (ATR, FT) *ṽ* (cm⁻¹) 2961 (vw), 2930 (vw), 2897 (vw), 2837 (vw), 1730 (vw), 1686 (vw), 1628 (m), 1612 (s), 1582 (s), 1524 (m), 1458 (w), 1427 (w), 1317 (w), 1302 (w), 1238 (s), 1157 (m), 1113 (w), 991 (vs), 947 (s), 914 (s), 874 (m), 827 (m), 797 (s), 733 (m), 704 (m), 656 (w). **Elemental Anal.** Calcd. for C₁₀H₈N₂S₂: C, 54.52; H, 3.66; N, 12.72. Found: C, 54.32; H, 3.97; N, 12.20.

### Example 22: 2H,6H,10H-benzo[1,2-e:3,4-e':5,6-e"]tris([1 ,3]thiazine) (1y-S)

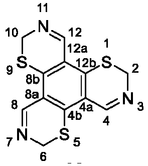

Compound **2y-S** was used as starting material. Compound **1y-S** was purified by flash chromatography using EtOAc/MeOH (20:1 and 10:1 v/v) and obtained in 4% yield (53 mg, 0.18 mmol) as a brown solid.

**Retention factor:** 0.41 (EtOAc/MeOH 10:1 v/v). **Melting point:** 217 °C (dec.). **¹H NMR:** (400 MHz, DCM-d₂) *δ* (ppm) 8.47 (t, *J* = 1.8 Hz, 3H, *H*4,8,12), 4.72 (d, *J* = 1.8 Hz, 6H, *H*2,6,10). **¹³C{¹H} NMR:** (100 MHz, DCM-d₂) *δ* (ppm) 156.9 (*C*4,8,12), 143.2 (*C*4b,8b,12b), 121.5 (*C*4a,8a,12a), 48.0 (*C*2,6,10). **HRMS (+APCI):** [M+H]⁺: *m*/*z* calcd. for C₁₂H₁₀N₃S₃⁺. 292.0031, found: 292.0031. **IR:** (ATR, FT) *ṽ* (cm⁻¹) 2970 (vw), 2907 (w), 2839 (vw), 1742 (w), 1672 (w), 1622 (s), 1514 (vs), 1423 (w), 1381 (w), 1339 (w), 1269 (w), 1236 (w), 1209 (w), 1194 (w), 1161 (w), 1128 (w), 1032 (vs), 953 (s), 910 (m), 816 (m), 750 (m), 717 (m), 638 (m).

As shown by the above examples, the compounds represented by the formula (1) can be obtained in high yields and with extensive substrate scope. Even annulated *π* systems like thionaphthols **2v-S** and **2w-S** were suitable for thiazine formation with high yields.

### Example 23: 8-Methyl-2H-benzo[e][1,3]thiazine-2,4(3H)-dione

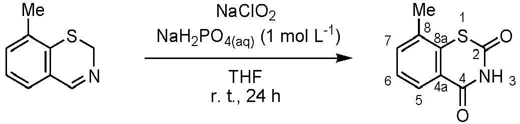

A 100 mL round-bottomed flask was filled with sodium chlorite (2.4 g, 80 wt%, 20 mmol), aqueous sodium dihydrogenphosphate solution (6.3 mL, 1 mol L⁻¹, 6.3 mmol) and THF (16.3 mL). A solution of 8-methyl-2*H*-benzo[e][1,3]thiazine **1b-S** (682 mg, 4 mmol) in THF (4.2 mL) was added dropwise and the resulted slightly yellow emulsion was stirred vigorously at room temperature for 24 h. Afterwards, the emulsion was mixed with EtOAc (120 mL) and washed with water (40 mL), aqueous sodium thiosulfate solution (40 mL, 1 mol L⁻¹) and brine (40 mL). The organic phase was dried using Na₂SO₄. After filtration the solvent of the filtrate was evaporated in vacuo and purified by flash chromatography using n-pentane/EtOAc (2:1 v/v) as solvent. After dissolving in ethyl acetate and precipitating with n-pentane the product was obtained as colourless solid in 22% yield (181 mg, 0.9 mmol).

**Retention factor:** 0.57 (n-pentane/EtOAc2:1 v/v). **Melting point:** 221 -224 °C. **¹H NMR:** (600 MHz, DMSO-d₆) *δ* (ppm) 12.46 (s, 1H, -NH-), 8.09 (d, *J* = 7.9 Hz, 1H, H5), 7.62 (d, *J =* 7.4 Hz, 1H, H7), 7.41 (t, *J =* 7.7 Hz, 1H, H6), 2.31 (s, 3H, -C*H*₃). ¹³C{¹H} NMR: (150 MHz, DMSO-d₆) *δ* (ppm) 163.9 (C4), 163.1 (C2), 135.3 (C7), 133.6 (C8), 132.8 (C8a), 127.9 (C5), 126.8 (C6), 122.7 (C4a), 18.6 (-CH₃). **HRMS (+EI):** [M]⁺: *m*/*z* calcd. for C₉H₇NO₂S⁺: 193.0192, found: 193.0191. IR: (ATR, FT) *ṽ* (cm⁻¹) 3153 (w), 3101 (vw), 3040 (w), 2835 (w), 1651 (vs), 1578 (s), 1447 (m), 1356 (s), 1298 (m), 1271 (m), 1231 (s), 1177 (w), 1159 (w), 1103 (w), 1057 (w), 1030 (w), 1001 (w), 908 (w), 837 (m), 810 (s), 779 (m), 746 (s), 675 (vs), 633 (m). **Elemental Anal.** Calcd. for C₉H₇NO₂S: C, 55.95; H, 3.65; N, 7.25. Found: C, 55.93; H, 3.81; N, 7.25.

For obtaining the compound having the formula (4) on the basis of 8-Methyl-2*H-*benzo[e][1,3]thiazine-2,4(3*H*)-dione the remaining steps depicted in Figure 2 can be carried out.

## Claims

1. A method for producing a compound represented by the following general formula (1), 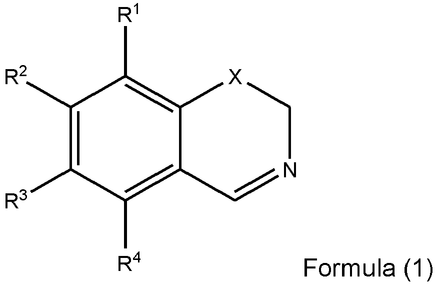 wherein the method comprises reacting a compound represented by the following general formula (2) 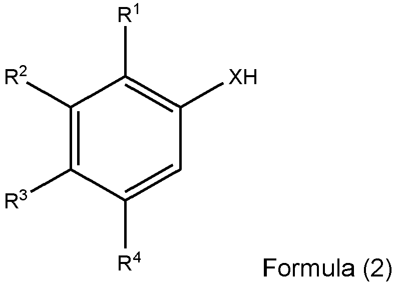
with hexamethylenetetramine in the presence of an acid,
wherein X is S or Se, and
R¹ to R⁴ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, a halogen atom, -XH, -S-alkyl, -NE¹E², -NO₂, -CN, -OE³, -C(O)E⁴, -C(O)NE⁵E⁶, -COOE⁷, and -SO₃E⁸, wherein E¹ to E⁸ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, wherein two or more of R¹ to R⁴ may bind to each other to form one or more rings.

2. The production method according to claim 1, wherein X is S.

3. The production method according to claim 1 or 2, wherein R¹ to R⁴ are each independently selected from the group consisting of a hydrogen atom, a substituted or unsubstituted alkyl group, a substituted or unsubstituted alkenyl group, a halogen atom, -XH, -S-alkyl, -NO₂, -OE³, and -COOE⁷, wherein two or more of R¹ to R⁴ may bind to each other to form one or more rings.

4. The production method according to any one of claims 1 to 3, wherein only one group -XH is present in the compound represented by the general formula (2).

5. The production method according to any one of claims 1 to 4, wherein at least two of R¹ to R⁴ are a hydrogen atom.

6. The production method according to any one of claims 1 to 5, wherein the acid is selected from the group consisting of trifluoroacetic acid, p-toluenesulfonic acid, trifluoromethanesulfonic acid, methanesulfonic acid, and acetic acid.

7. The production method according to any one of claims 1 to 6, wherein the acid is present in an amount of at least 10 equivalents in relation to the amount of groups -XH present in the compound represented by the general formula (2).

8. The production method according to any one of claims 1 to 7, wherein hexamethylenetetramine is present in an amount of at least 1.0 equivalents in relation to the amount of groups -XH present in the compound represented by the general formula (2).

9. The production method according to any one of claims 1 to 8, wherein the step of reacting is carried out at a temperature of from 0°C to 140°C.

10. The production method according to any one of claims 1 to 9, wherein the step of reacting is carried out for a duration of from 30 s to 10 d.

11. The production method according to any one of claims 1 to 10, wherein the step of reacting is carried out in the presence of an inert and/or dry atmosphere.

12. The production method according to any one of claims 1 to 11, wherein the compound of the general formula (1) is selected from the compounds of the following formulas (1a) to (1y)

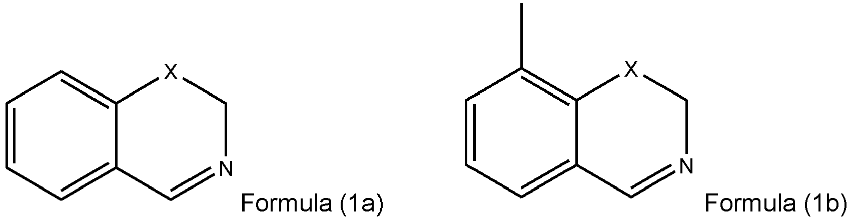

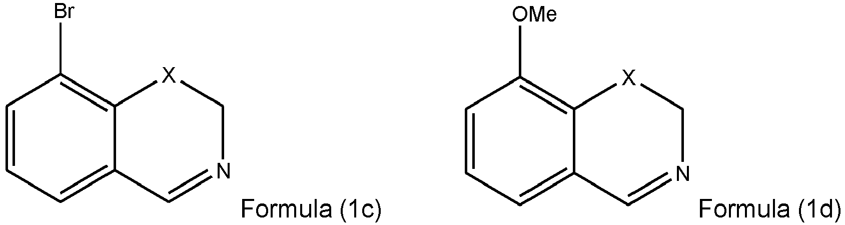

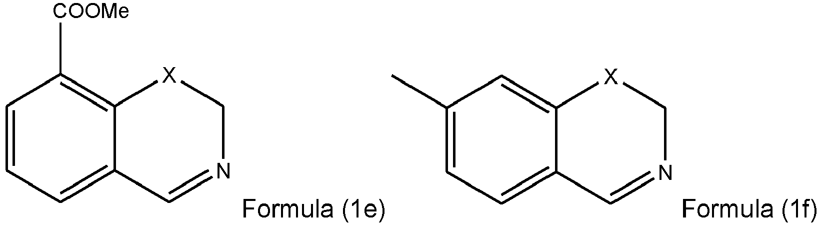

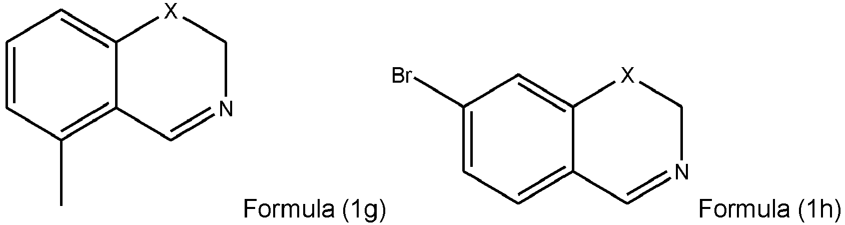

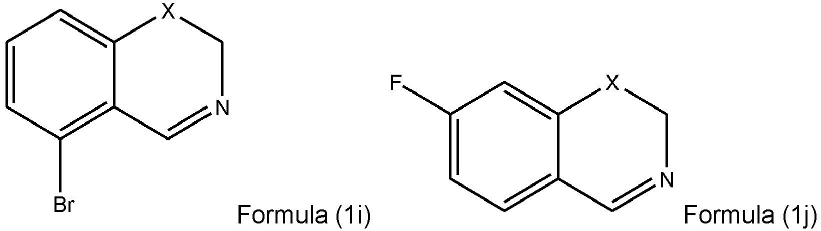

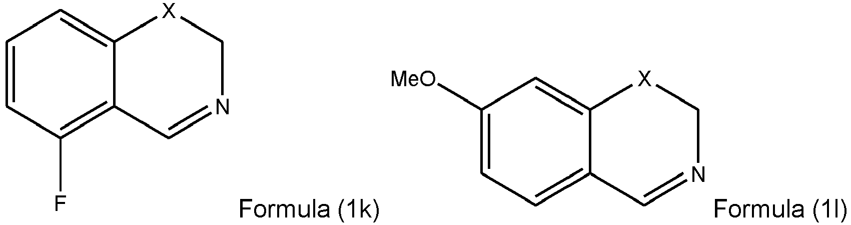

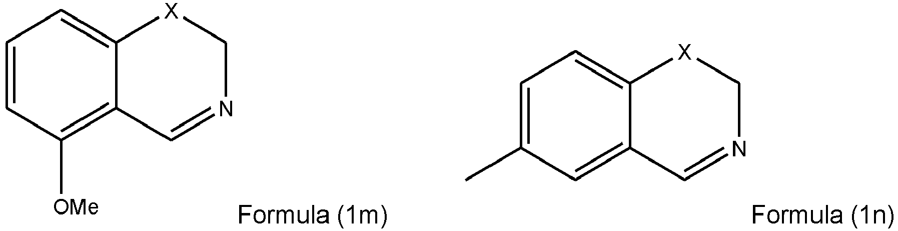

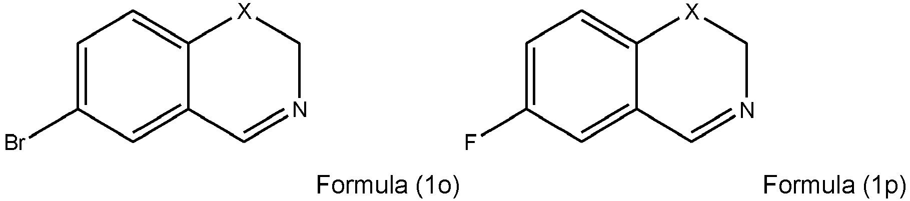

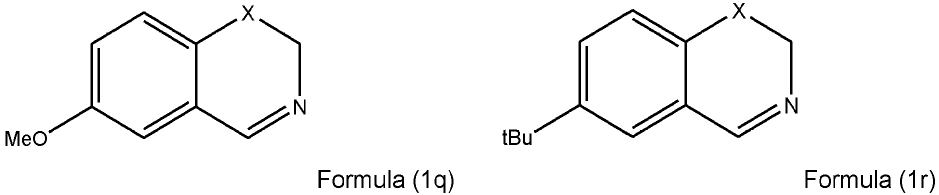

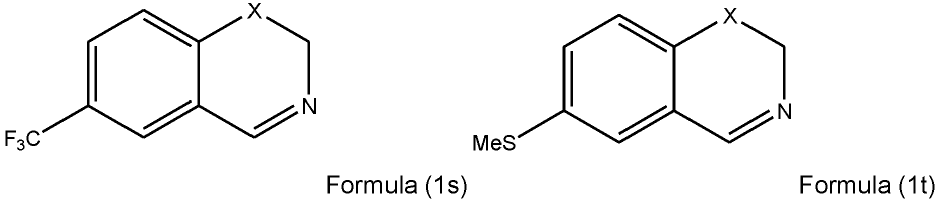

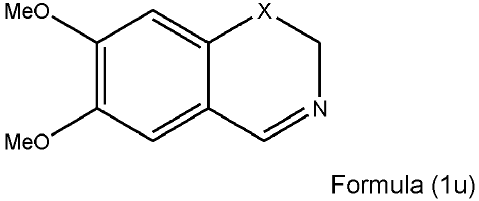

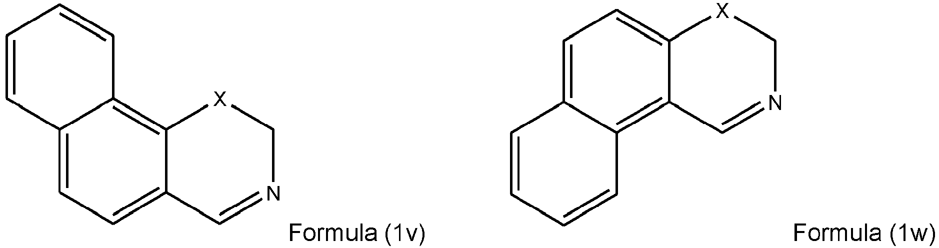

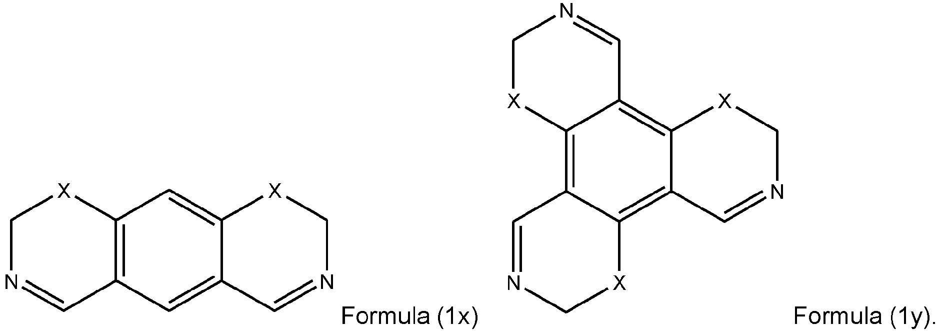

13. A method for producing a compound represented by the following general formula (3), 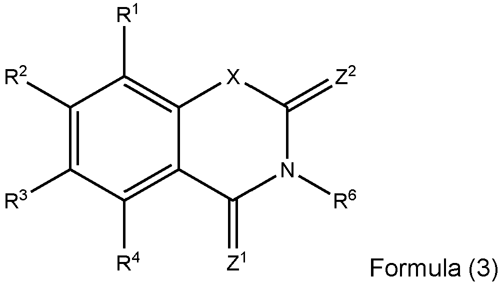
wherein the method comprises carrying out the production method according to any one of claims 1 to 12 to produce the compound of the general formula (1), and converting the compound of the general formula (1) into the compound of the general formula (3),
wherein X and R¹ to R⁴ are defined as above,
wherein Z¹ and Z² are each independently selected from O or NR⁵,
wherein R⁵ is selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, wherein R⁶ is selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, and a substituted or unsubstituted amino group, and
wherein R⁵ and R⁶ may bind to each other to form one or more rings.

14. The production method according to claim 13, wherein the compound of the general formula (3) is a compound of the formula (4)

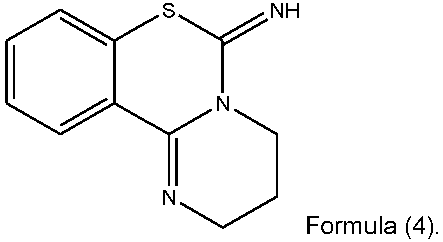

15. Use of hexamethylenetetramine and an acid for converting a thiol or selenol group and an adjacent CH group in an aromatic group into a 2-H-[1,3]-thiazine or 2-H-[1,3]-selenazine.
